# EUROPEAN PATENT SPECIFICATION

(11) **EP 1 869 073 B1**
(45) Date of publication and mention of the grant of the patent: **17.08.2011**
(21) Application number: 06724635.5
(22) Date of filing: 07.04.2006
(51) Int. Cl.: C07K 14/435

(54) **POLYPEPTIDES OF LEISHMANIA MAJOR AND POLYNUCLEOTIDES ENCODING SAME AND VACCINAL, THERAPEUTICAL AND DIAGNOSTIC APPLICATIONS THEREOF**
POLYPEPTIDE VON LEISHMANIA MAJOR UND DAFÜR CODIERENDE POLYNUKLEOTIDE, UND DEREN ANWENDUNGEN ALS IMPFSTOFFE, THERAPEUTIKA UND DIAGNOSTIKA
POLYPEPTIDES DE LEISHMANIA MAJOR, POLYNUCLEOTIDES CODANT CES POLYPEPTIDES, ET LEUR APPLICATIONS A DES FINS VACCINALES, THERAPEUTIQUES OU DIAGNOSTIQUES

(30) Priority: 08.04.2005 CA 2503932; 05.04.2006 CA 2540736
(43) Date of publication of application: 26.12.2007
(73) Proprietor: INSTITUT PASTEUR, 75724 Paris Cedex 15 (FR); INSTITUT PASTEUR DE TUNIS, 1002 Tunis Belvedere (TN)
(72) Inventor: CHENIK, Mehdi, TN-2070 La Marsa (TN); LAKHAL, Sami, TN-Tunis (TN); LOUZIR, Hechmi, TN-2070 La Marsa (TN); DELLAGI, Koussay, 1002 Tunis Belvedere (TN)
(74) Representative: Desaix, Anne
(86) International application number: PCT/EP2006/003978
(87) International publication number: WO 2006/108720

(56) References cited:
- FR-A- 2 826 018
- DATABASE EMBL [Online] 21 June 2005 (2005-06-21), "Leishmania major chromosome 5 strain Friedlin, complete sequence LmjF14_01_20050601_V5.2" XP002400607 retrieved from EBI accession no. EM_HTG:CT005253 Database accession no. CT005253 -& IVENS ALASDAIR C ET AL: "The genome of the kinetoplastid parasite, Leishmania major" SCIENCE (WASHINGTON D C), vol. 309, no. 5733, July 2005 (2005-07), pages 436-442,416, XP002400473 ISSN: 0036-8075

## Description

### FIELD OF THE INVENTION

The invention relates to new proteins of *Leishmania major* and to therapeutical and diagnostic applications thereof. More particularly, the invention relates to excreted/secreted polypeptides and polynucleotides encoding same, compositions comprising the same, and methods of diagnosis, vaccination and treatment of Leishmaniasis.

### BRIEF DESCRIPTION OF THE PRIOR ART

The leishmaniases are a heterogeneous group of diseases that affect millions of people in tropical and subtropical areas of the world [Desjeux, 1996]. Depending on *Leishmania* species involved and the immunological status of the human host, the disease ranges from asymptomatic infections to self-limiting cutaneous lesion(s) or fatal visceral forms. During their life cycle, parasites alternate between two stages: flagellated promastigotes in the midgut of the insect vector and amastigotes in the host macrophage [Alexander, 1992; Handman, 1999]. At this later stage, *Leishmania* parasites are sequestered and resist in the phagolysosome, originated from the fusion of phagosomes with lysosomes [Handman, 1999; Duclos and Desjardins, 2000; Sacks, 2001; Amer and Swanson, 2002; Cunningham, 2002].

Over the past decades, several molecules playing a key role either in the biology of the parasite or as target for antibody or cellular responses have been identified. Previous observations indicate that excreted molecules from intracellular pathogens such as *Mycobacterium tuberculosis* and *Toxoplasma gondii* contain antigens that are highly immunogenic and protective in vaccine models [Prigione, 2000; Mustafa, 2002; Daryani, 2003; Pym, 2003; Shams, 2004]. Similarly, *Leishmania* promastigote culture filtrate proteins also elicit strong immunity and protection in *L. major* BALB/c infection [Webb, 1998; Mendez, 2002]. However, there is no data on the secreted/excreted molecules from the amastigote stage of the parasite, the invading form that disseminate in the mammalian host. Due to their location, antigens secreted/excreted by *Leishmania* amastigotes are of high importance, partly due to their capacity to generate peptides that can be loaded onto CMH class I or II molecules and may serve as an interesting target for cellular immune responses. On the contrary, molecules released into the *Leishmania* phagosome may also subvert the presentation machinery associated with endoplasmic reticulum-mediated phagocytosis, which may represent an immune evasion strategy to avoid cellular immune response.

In an effort to attempt to identify new secreted/excreted molecules of *Leishmania major* parasite, we used culture supernatants of stationary phase promastigotes cultivated during 8 hours in serum-free medium, at pH and temperature that mimic the phagosome conditions, to immunize mice. Immune sera were then used to screen a cDNA expression library of *L. major.* A total of 34 different clones were isolated and sequenced. 21 percent of molecules exhibit significant sequence homology to a group of secreted proteins. The others are not described and constitute new sequences. Some of these proteins are logical candidates for analysis as potential vaccine components or drug targets.

There is therefore a need in the art for new HIV treatments or vaccines that elicit a broad, long-lasting and neutralizing immune response. There is also a need to provide for new diagnostic and immunomonitoring methods with regards to HIV infections.

### SUMMARY

The invention satisfies at least one of the above-mentioned needs.

More specifically, an object of the invention concerns an isolated polynucleotide comprising a sequence encoding an excreted/secreted polypeptide of *Leishmania major,* said sequence comprising a nucleotide sequence substantially identical to the sequence of SEQ ID NO: 1

Other objects of the invention concern an isolated or purified excreted/secreted polypeptide of *Leishmania major,* said polypeptide comprising an amino acid sequence substantially identical to the sequence of SEQ ID NO: 35

Still another object of the invention is to provide an immunogenic composition generating an immune response against a leishmaniasis, comprising a polynucleotide of the invention or a polypeptide of the invention, and an acceptable carrier.

Yet another object of the invention concerns a vaccine composition generating a protecting response against a leishmaniasis, comprising a polynucleotide of the invention or a polypeptide of the invenition, and an acceptable carrier.

Yet another object of the invention concerns an antibody obtainable by the immunization of an animal with a polypeptide of the invention.

Yet another object of the invention concerns an expression or a cloning vector containing a polynucleotide of the invention.

Yet another object of the invention concerns a method for preventing and/or treating a patient against an infection with a *Leishmania major* strain, the method comprising the step of administering to the patient a therapeutically effective amount of a composition of the invention or of an antibody of the invention

Yet another object of the invention concerns a method for identifying an excreted/secreted polypeptide of a *Leishmania major* strain, comprising *in vitro* cultivating Leishmania promastigotes under pH and temperature conditions naturally found in a host cell infected by a *Leishmania major* strain.

Yet another object of the invention concerns an *in vitro* diagnostic method for the detection of the presence or absence of antibodies indicative of a *Leishmania major* strain, which bind to a polypeptide of the invention to form an immune complex, comprising the steps of
a) contacting said polypeptide with a biological sample for a time and under conditions sufficient to form an immune complex; and
b) detecting the presence or absence of the immune complex formed in a).

A further object of the invention concerns a diagnostic kit for the detection of the presence or absence of antibodies indicative of a *Leishmania major* strain, comprising:
- a polypeptide of the invention;
- a reagent to detect polypeptide-antibody immune complex;
- optionally a biological reference sample lacking antibodies that immunologically bind with said peptide; and
- optionally a comparison sample comprising antibodies which can specifically bind to said peptide;
wherein said polypeptide, reagent, biological reference sample, and comparison sample are present in an amount sufficient to perform said detection.

A further object of the invention concerns an *in vitro* diagnostic method for the detection of the presence or absence of polypeptides indicative of a *Leishmania major* strain, which bind to an antibody of the invention to form an immune complex, comprising the steps of:
a) contacting said antibody with a biological sample for a time and under conditions sufficient to form an immune complex; and
b) detecting the presence or absence of the immune complex formed in a).

A further object of the invention concerns a diagnostic kit for the detection of the presence or absence of polypeptides indicative of a *Leishmania major* strain, comprising:
- an antibody of the invention;
- a reagent to detect polypeptide-antibody immune complex;
- optionally a biological reference sample lacking polypeptides that immunologically bind with said antibody; and
- optionally a comparison sample comprising polypeptides which can specifically bind to said antibody;
wherein said antibody, reagent, biological reference sample, and comparison sample are present in an amount sufficient to perform said detection.

A further object of the invention concerns a genetically modified Leishmania strain comprising at least one gene having a sequence comprising a nucleotide sequence substantially identical to the sequence of SEQ ID NO: 1, and wherein said at least one gene is underexpressed compared to a corresponding gene of a wild-type strain of Leishmania.

A further object of the invention concerns a genetically modified Leishmania strain comprising at least one gene having a sequence comprising a nucleotide sequence substantially identical to the sequence of SEQ ID NO: 1, and wherein said at least one gene is inactivated.

A further object of the invention concerns a method for detecting the presence or absence of lymphocytic stimulation in a subject suspected of Leishmaniasis, comprising the steps of:
a) obtaining a sample containing T Lymphocytes from said subject;
b) contacting the T lymphocytes with a polypeptide of the invention; and
c) detecting the presence or absence of a proliferative response of said T lymphocyte to the polypeptide.

A further object of the invention concerns a method for detecting the presence or absence of lymphocytic stimulation in a subject suspected of Leishmaniasis, comprising the steps of:
a) obtaining a sample containing T Lymphocytes from said subject;
b) contacting the T lymphocytes with a polypeptide of the invention; and
c) detecting the presence or absence of cytokines indicative of lymphocytic stimulation.

### BRIEF DESCRIPTION OF THE FIGURES

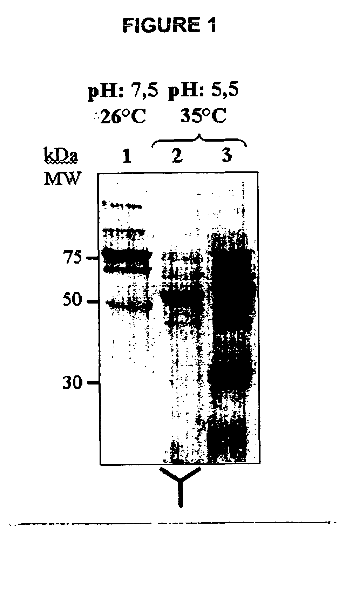

Figure 1 shows a SDS-PAGE illustrating excreted/secreted proteins of *Leishmania major* under different culture conditions.

### DETAILED DESCRIPTION OF THE INVENTION

The invention is directed to excreted/secreted polypeptides of *Leishmania major* and polynucleotide encoding same and their use in the preparation of compositions and vaccines. More specifically, the invention is concerned with compositions, vaccines and methods for providing an immune response and/or a protective immunity to mammals against a *Leishmania major* strain as well as methods for the diagnosis of a Leishmaniasis. The term "leishmaniasis" means an infection caused by any of the flagellate protozoans of the genus *Leishmania,* such as *Leishmania major.*

As used herein, the term "excreted/secreted polypeptide" of a *Leishmania major* strain refers to a polypeptide which is first synthetized into the parasite and then released into the extracellular medium by a secretion or excretion mechanism.

As used herein, the term "immune response" refers to the T cell response or the increased serum levels of antibodies to an antigen, or presence of neutralizing antibodies to an antigen, such as a *Leishmania major* protein. The term "immune response" is to be understood as including a humoral response and a cellular response.

The term "protection" or "protective immunity" refers herein to the ability of the serum antibodies and/or cellular response induced during immunization to protect (partially or totally) against Leishmaniasis caused by an infectious agent, such as *Leishmania major.* Thus, a mammal immunized by the compositions or vaccines of the invention will experience limited growth and spread of an infectious *Leishmania major.*

As used herein, the term "mammal" refers to any mammal that is susceptible to be infected by a *Leishmania major* strain. Among the mammals which are known to be potentially infected by *Leishmania major,* there are particularly humans.

### 1. Polynucleotides and polypeptides

The application describes an isolated polynucleotide comprising a sequence encoding an excreted/secreted polypeptide of *Leishmania major,* said sequence comprising a nucleotide sequence substantially identical to a sequence selected from the group consisting of SEQ ID NOS 1 to 34 and functional fragments thereof.

In an embodiment, the invention relates to an isolated polynucleotide comprising a sequence encoding an excreted/secreted polypeptide of *Leishmania major,* said sequence comprising a nucleotide sequence substantially identical to the sequence of SEQ ID NO: 1

As used herein, the term "functional fragment" refers to a polypeptide which possesses biological function or activity that is identified through a defined functional assay and which is associated with a particular biologic, morphologic, or phenotypic alteration in a cell or cell mechanism.

By the term "substantially identical", it is meant that the polynucleotide of the invention has a nucleic acid sequence which is at least 65% identical, more particularly 80 % identical and even more particularly 95% identical to any one of SEQ ID NO: 1 to 34.

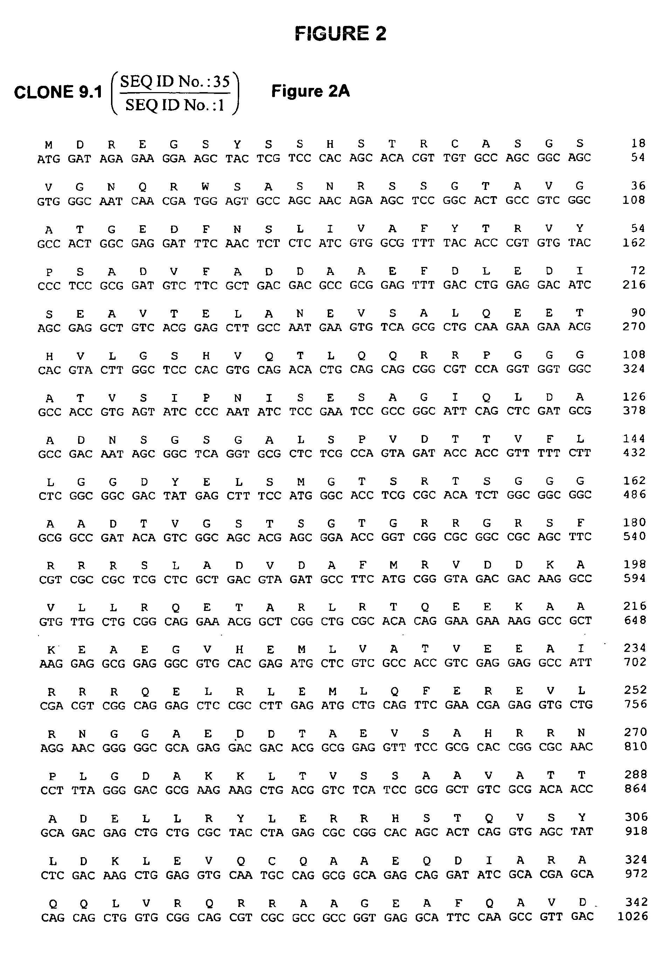

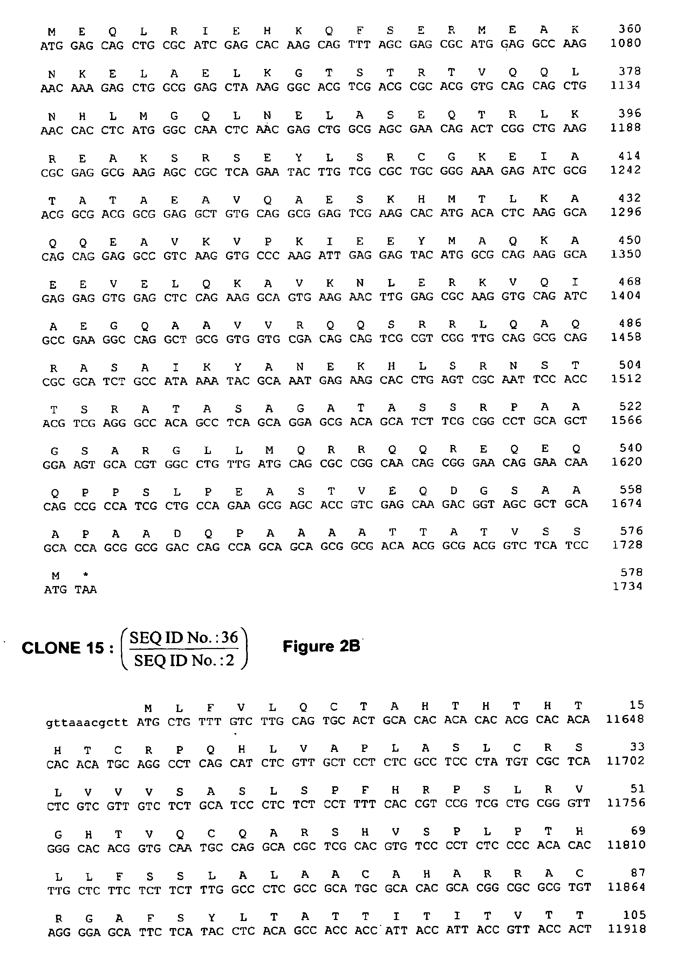

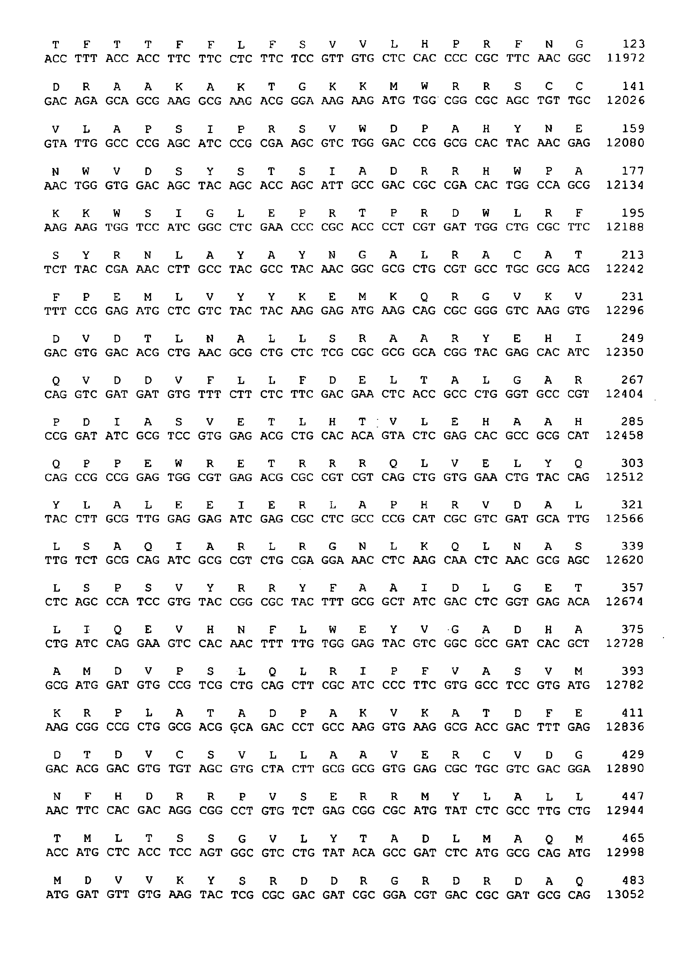

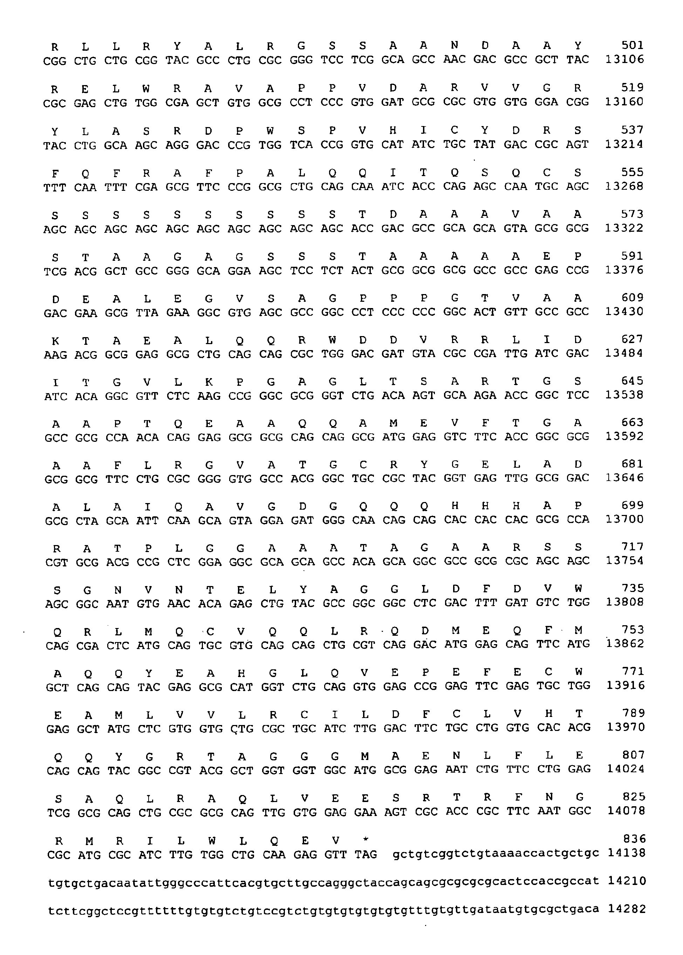

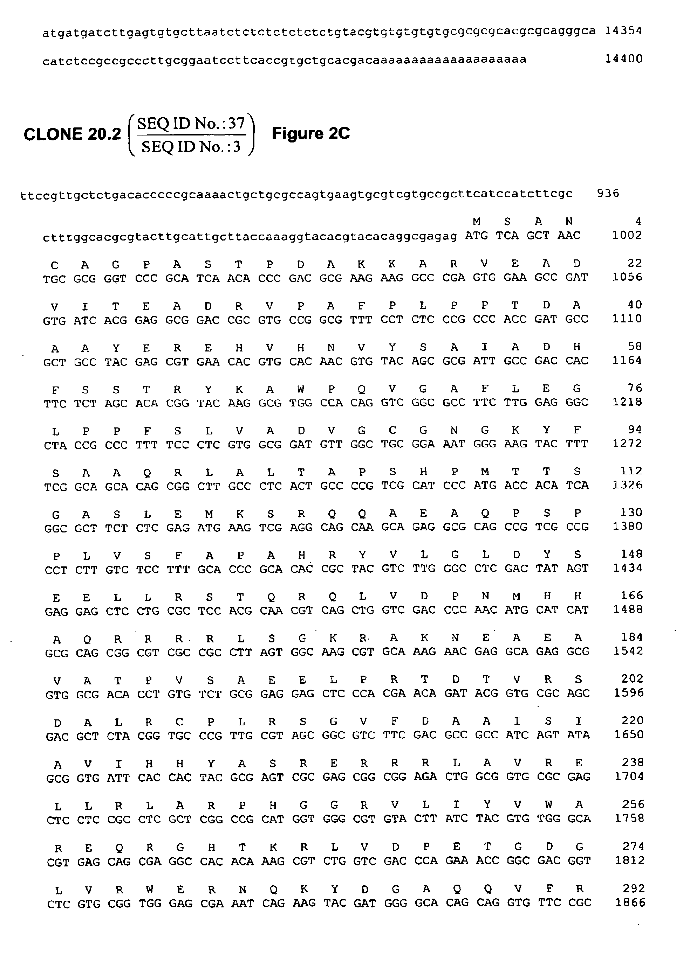

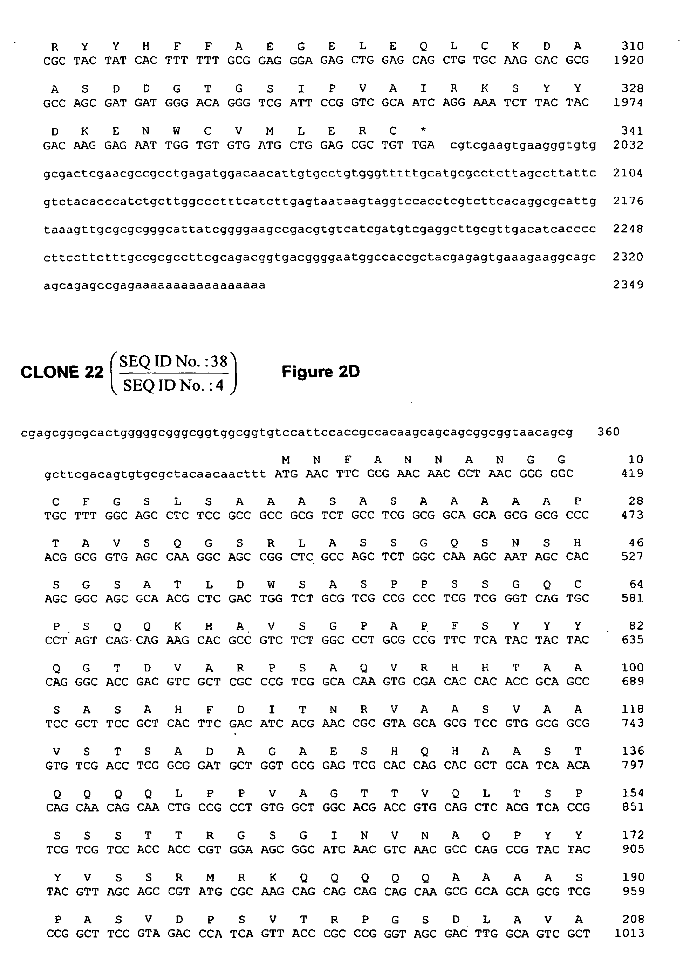

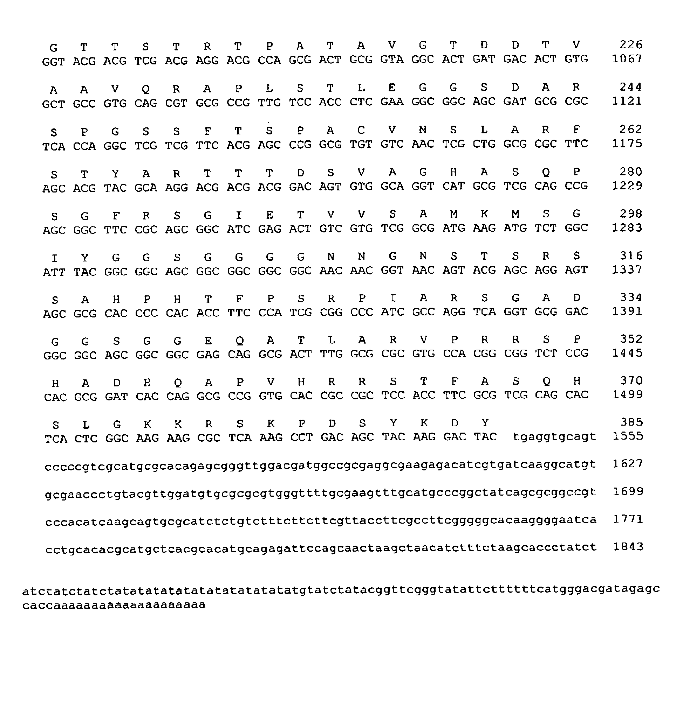

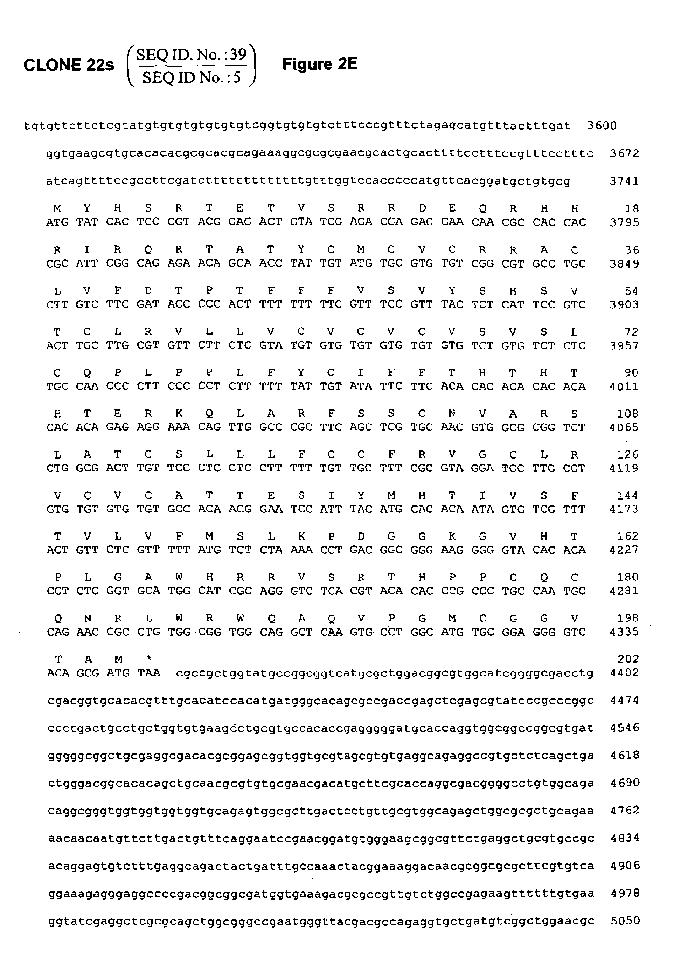

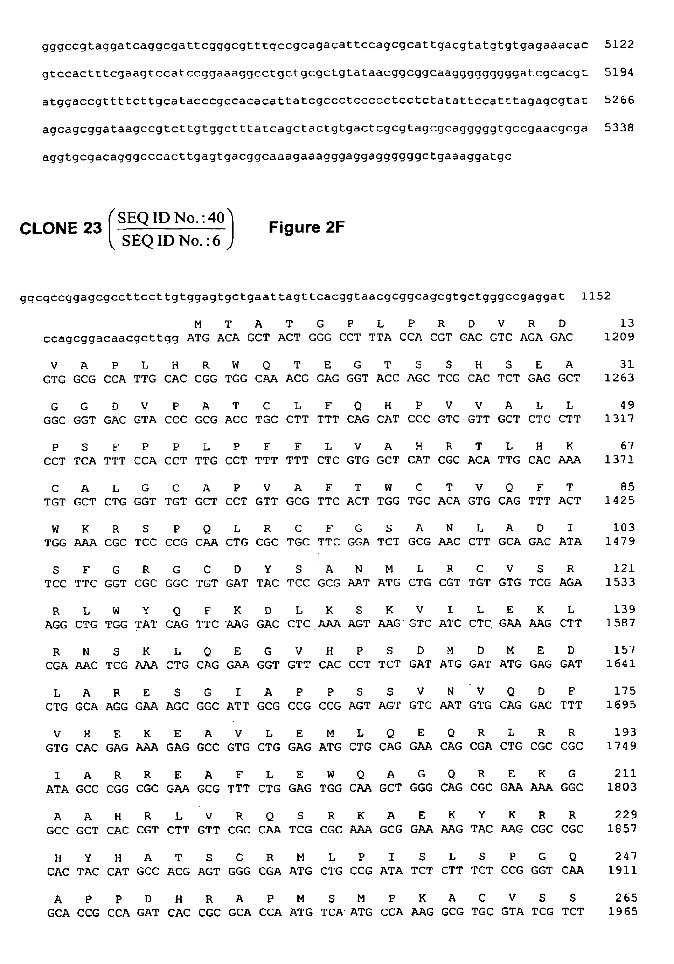

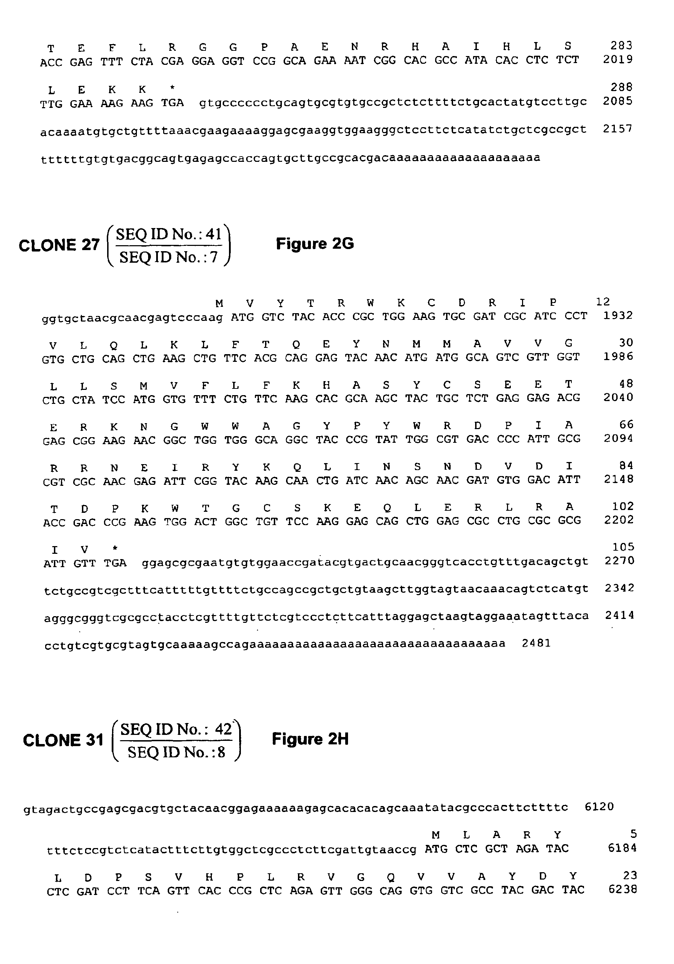

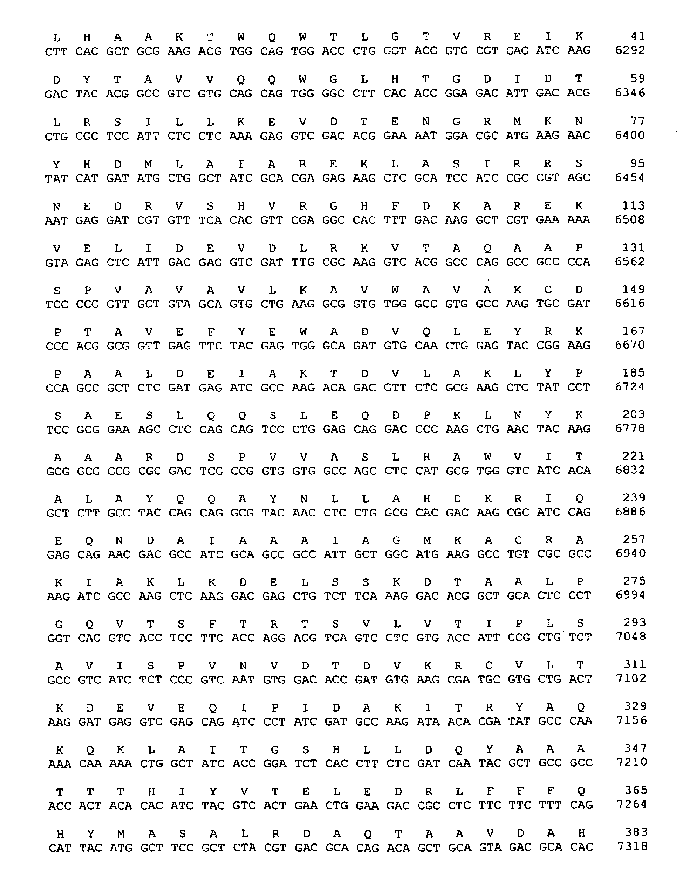

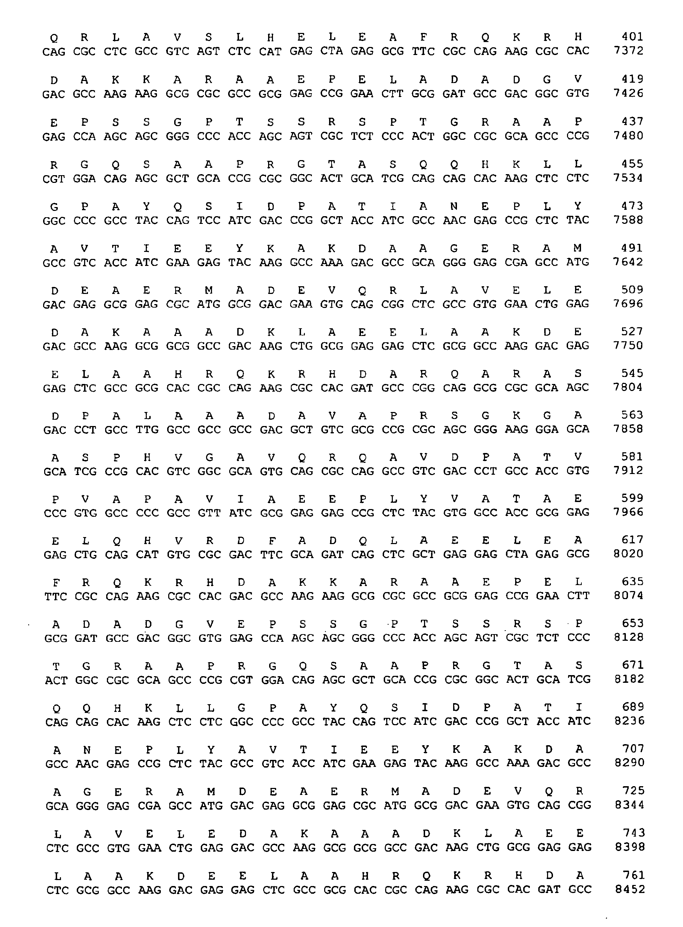

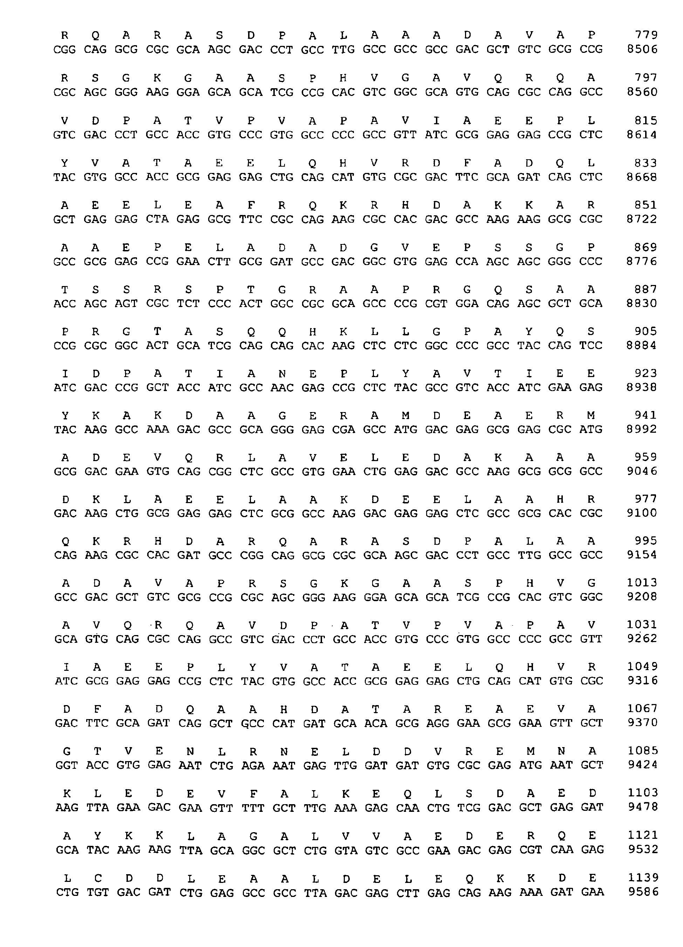

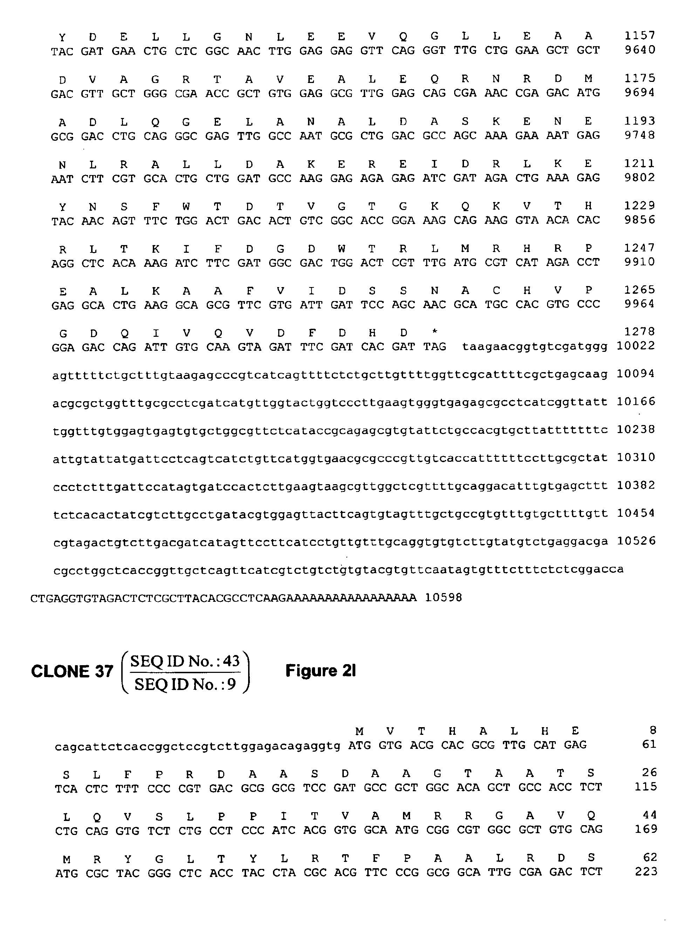

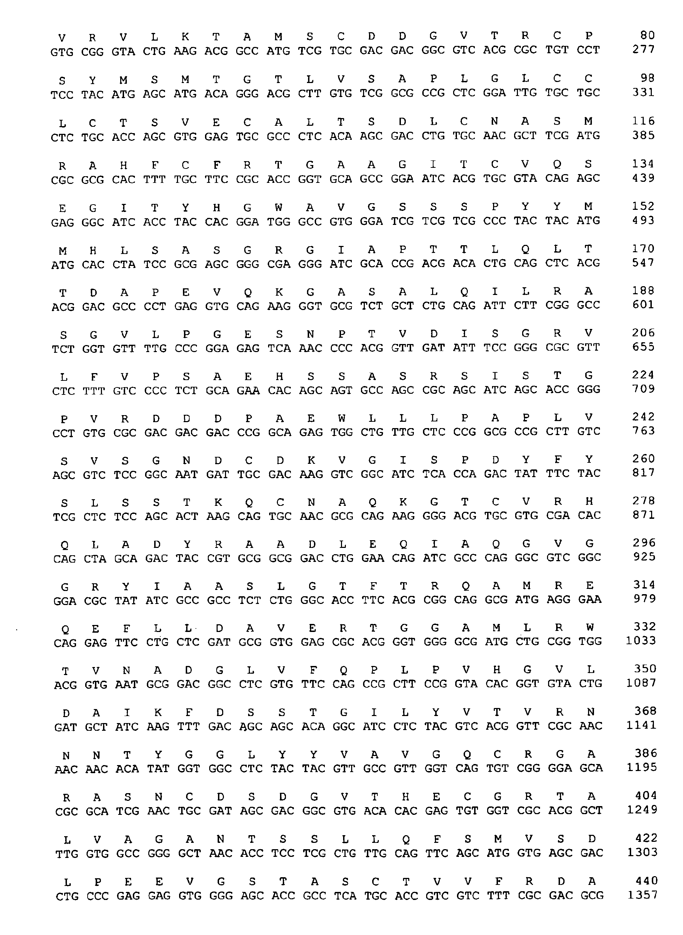

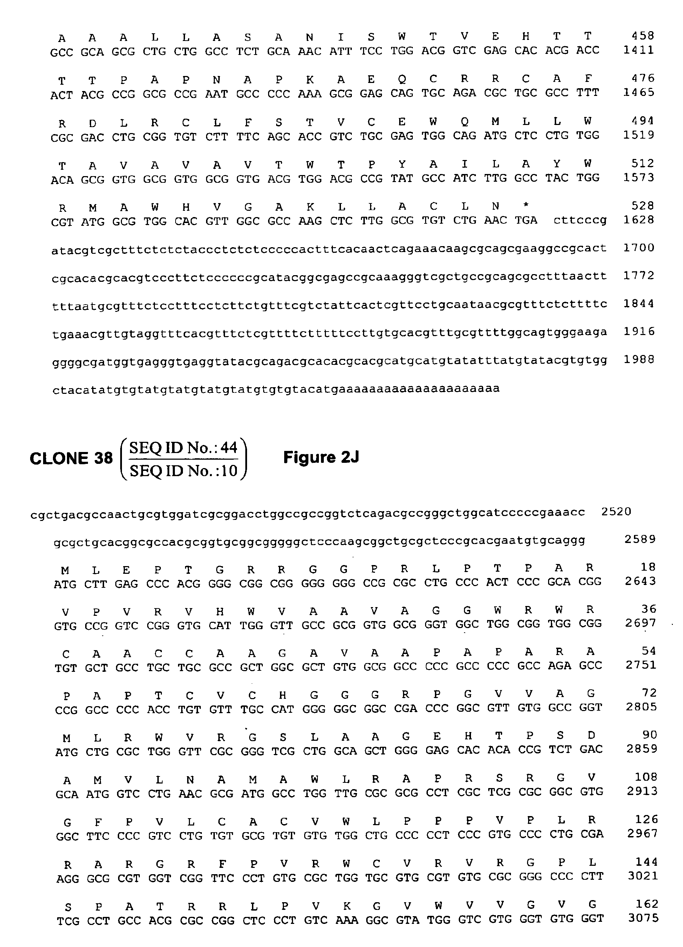

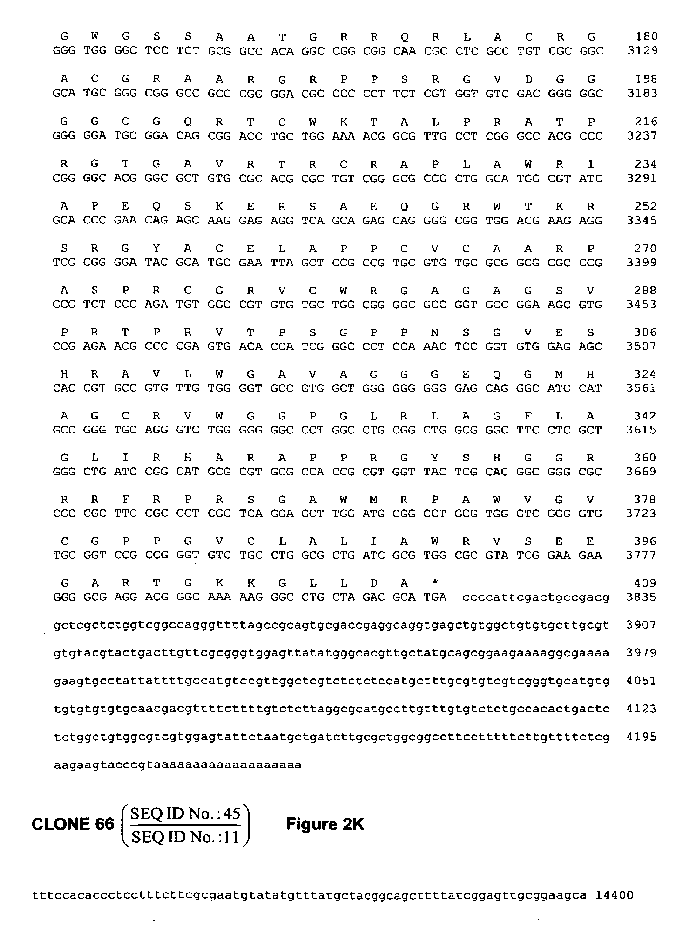

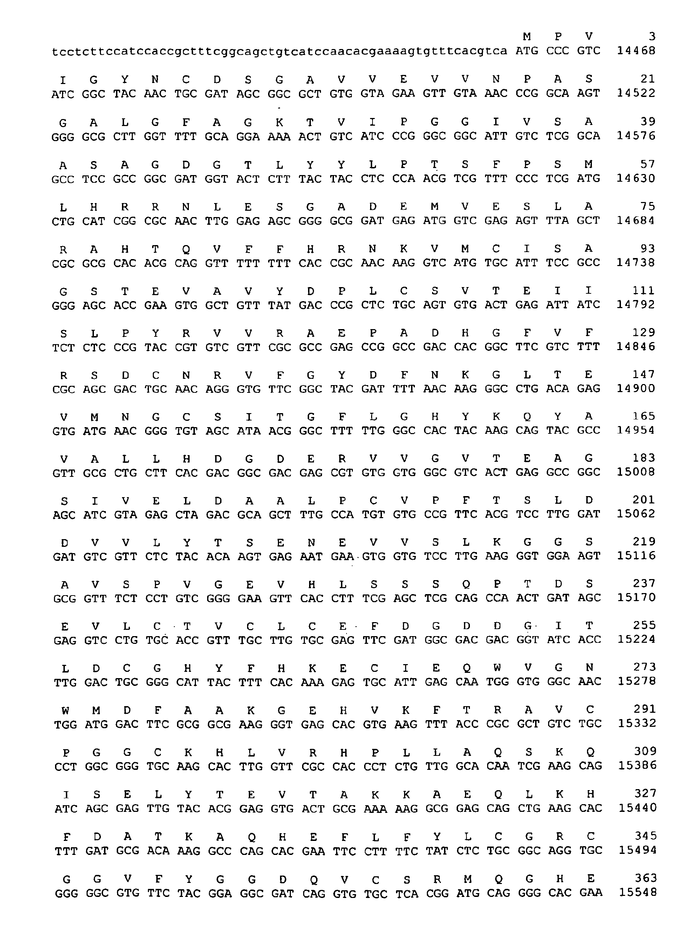

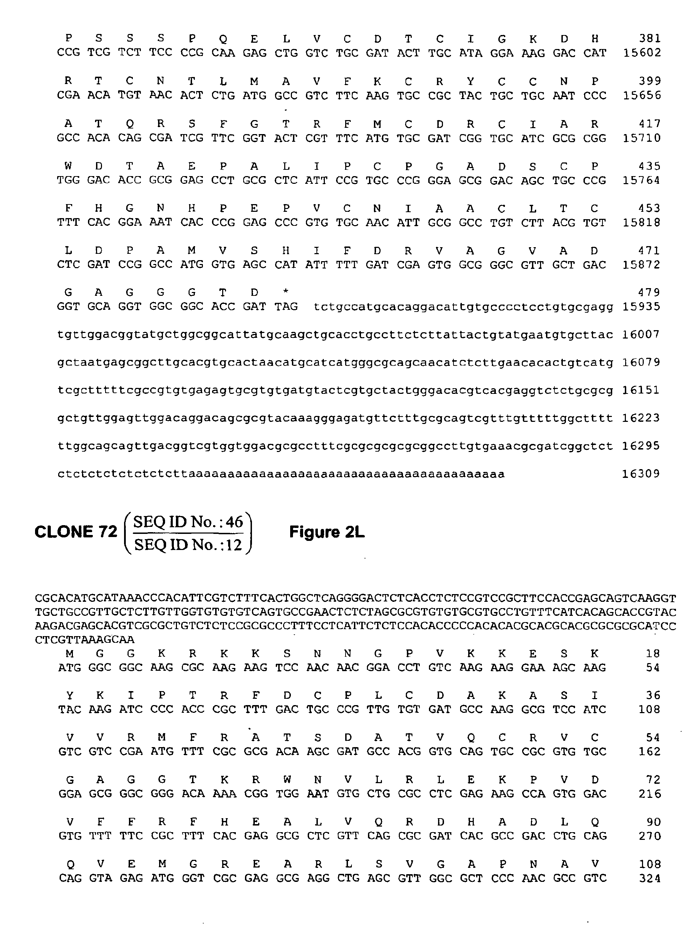

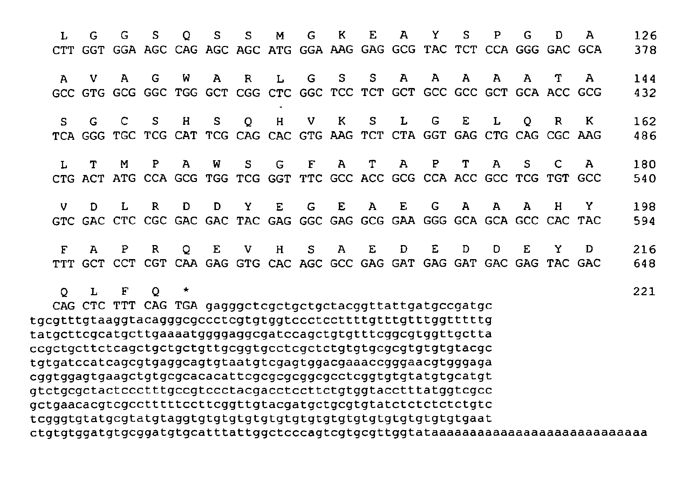

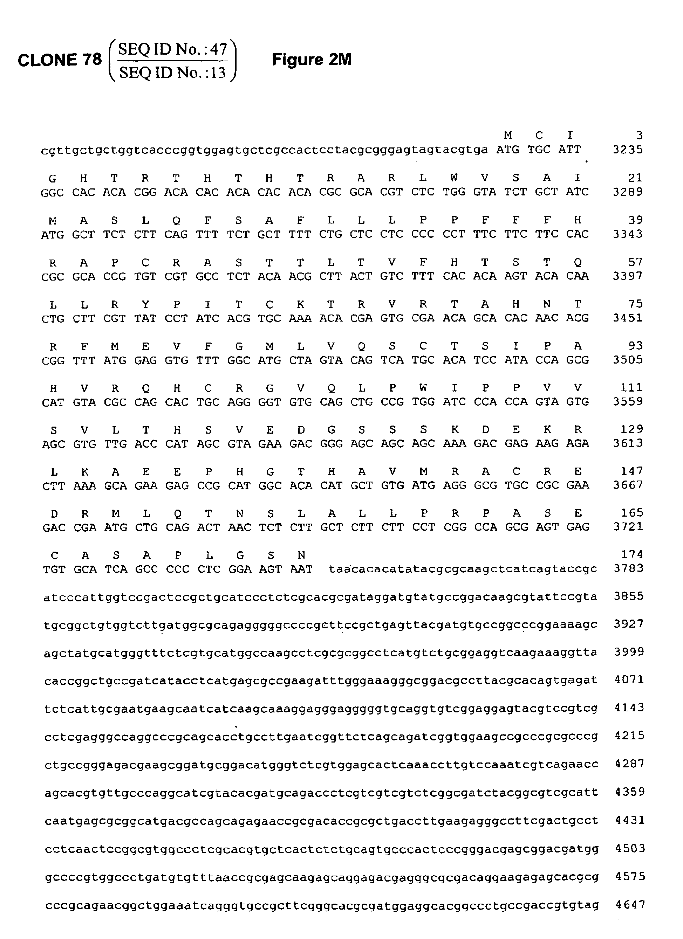

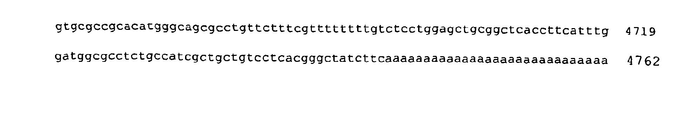

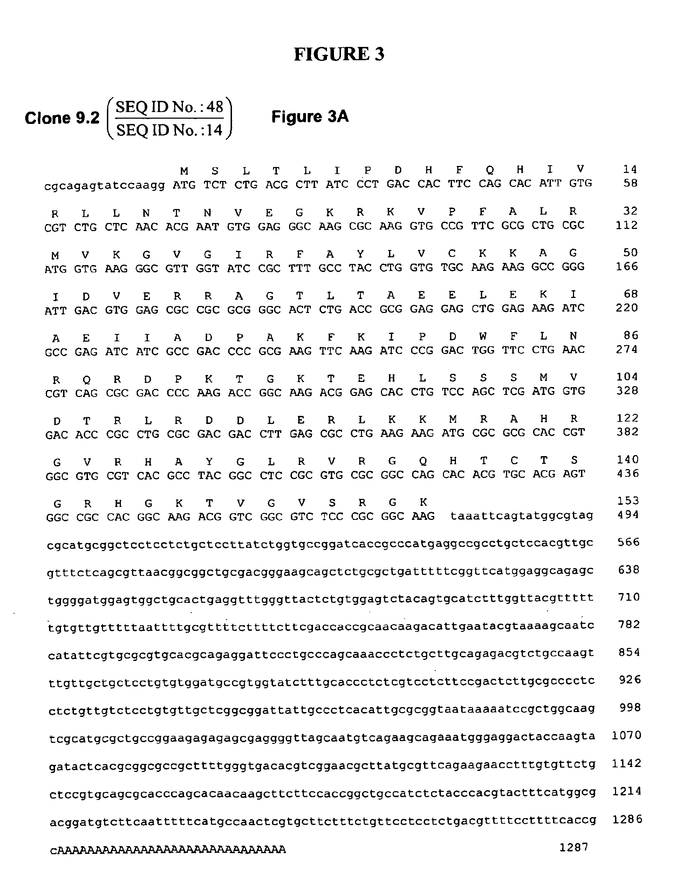

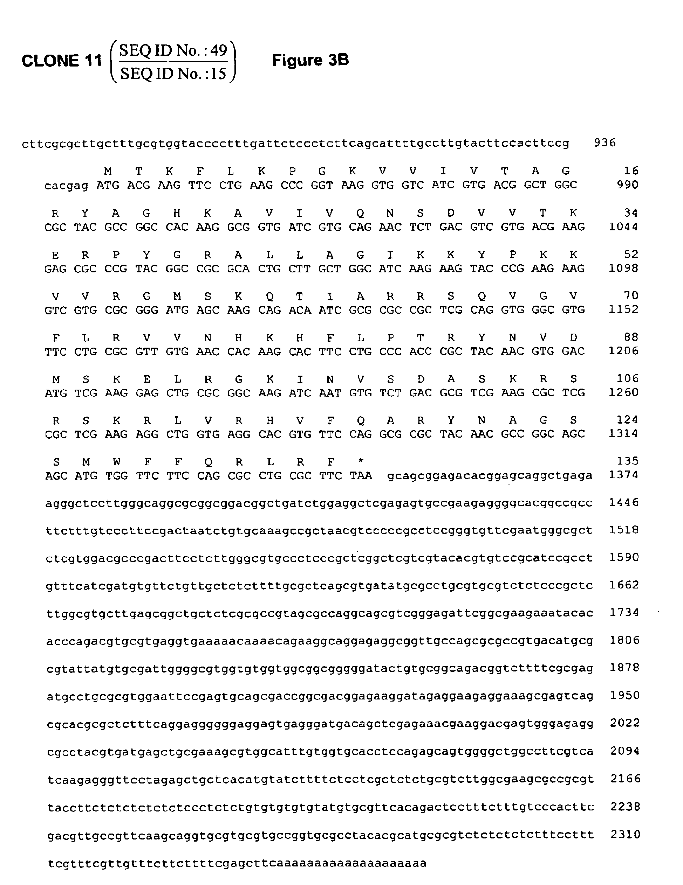

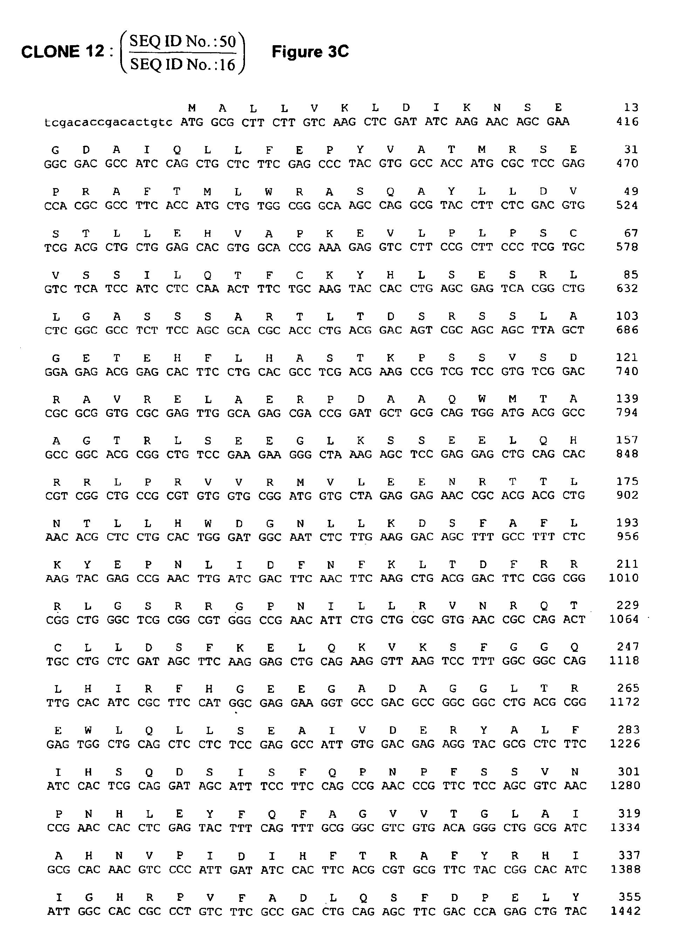

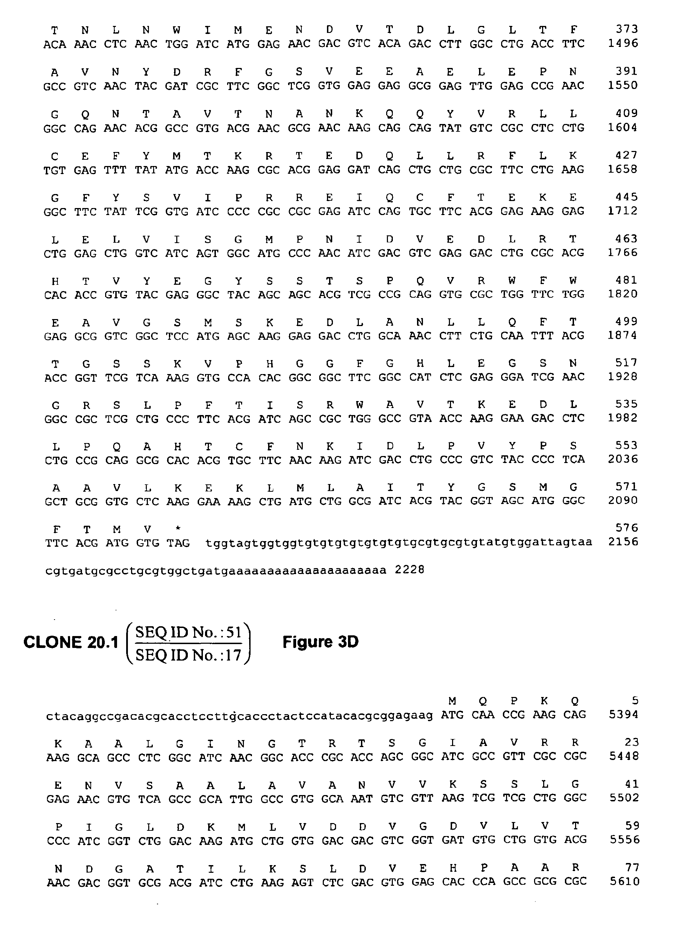

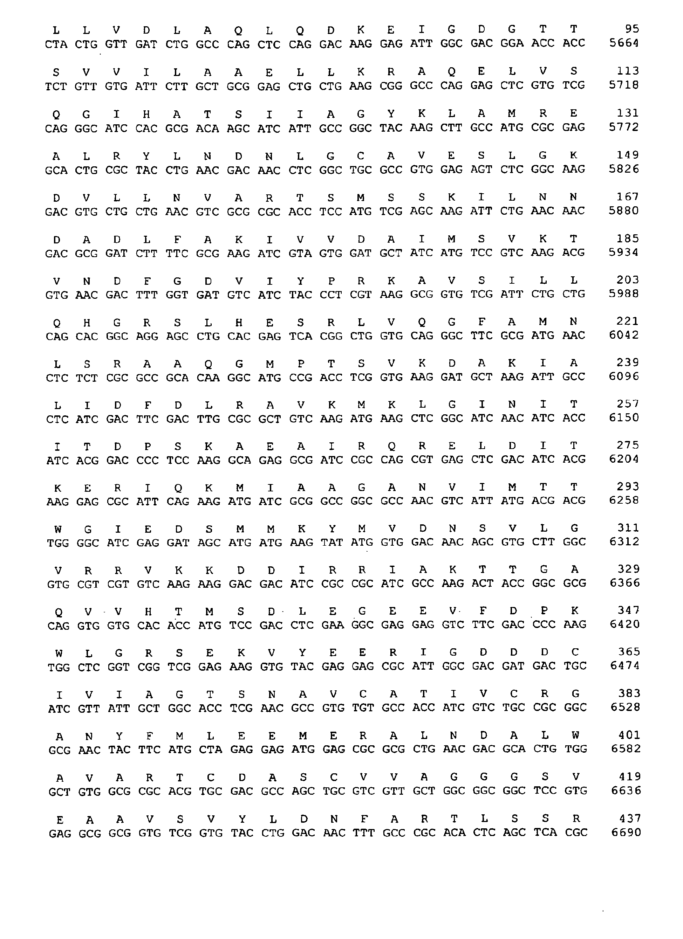

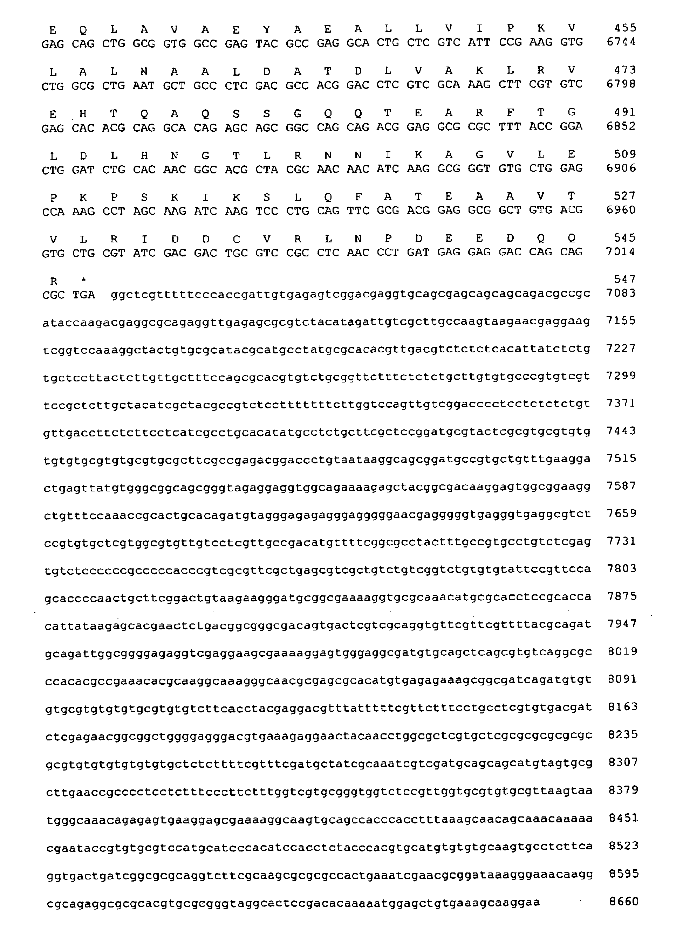

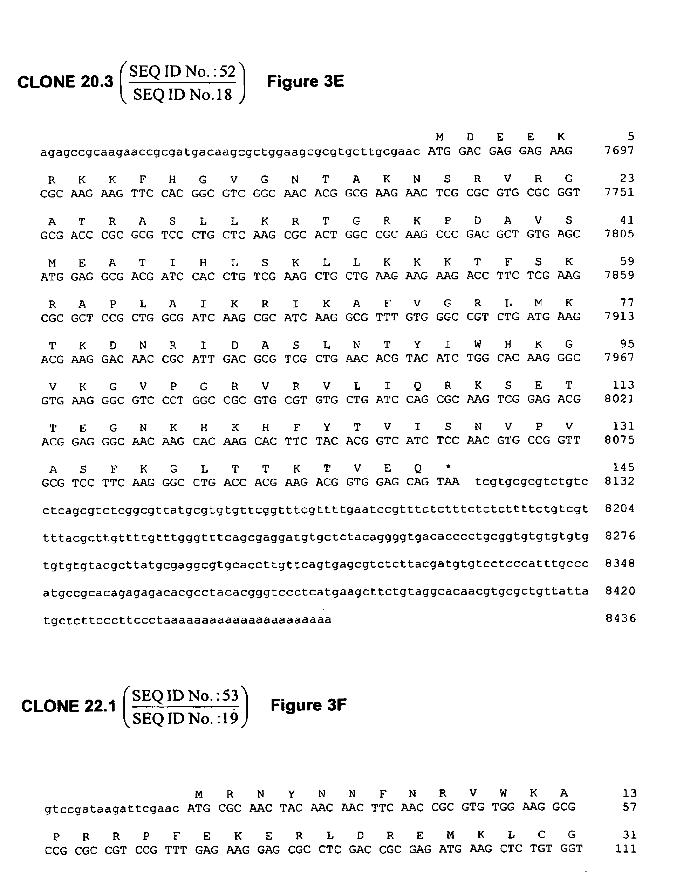

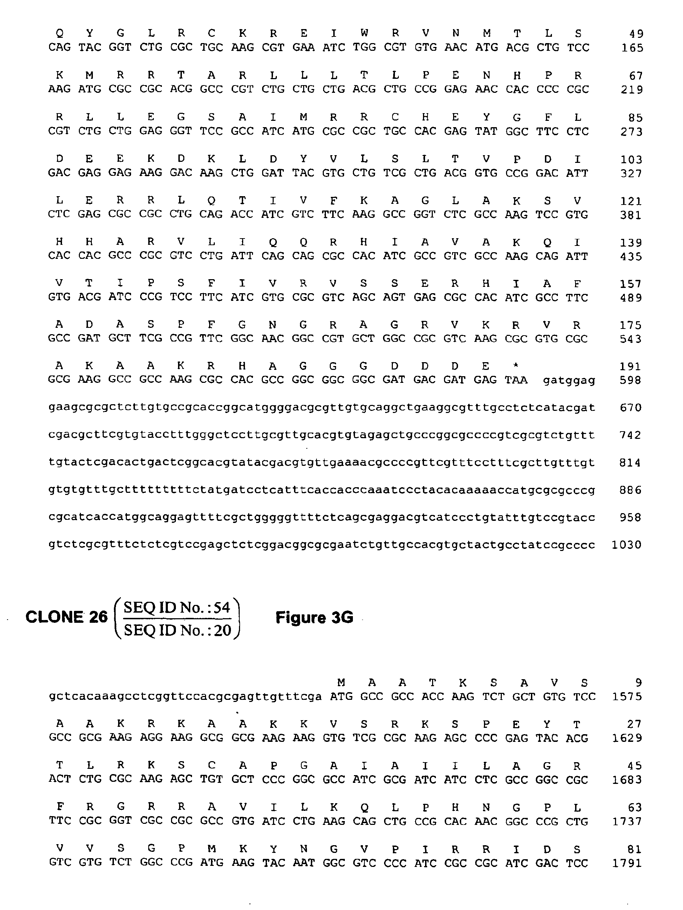

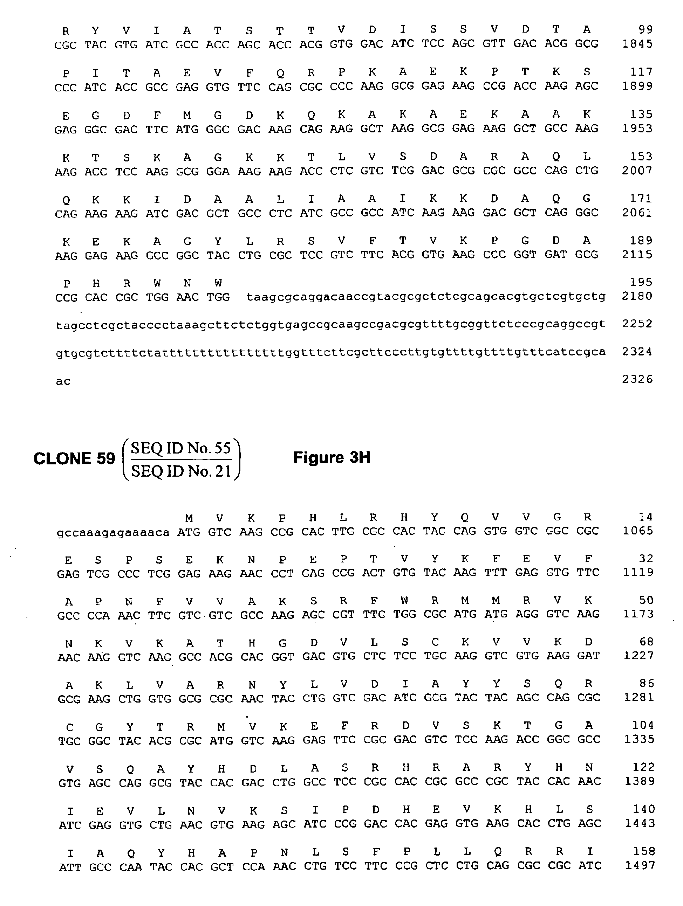

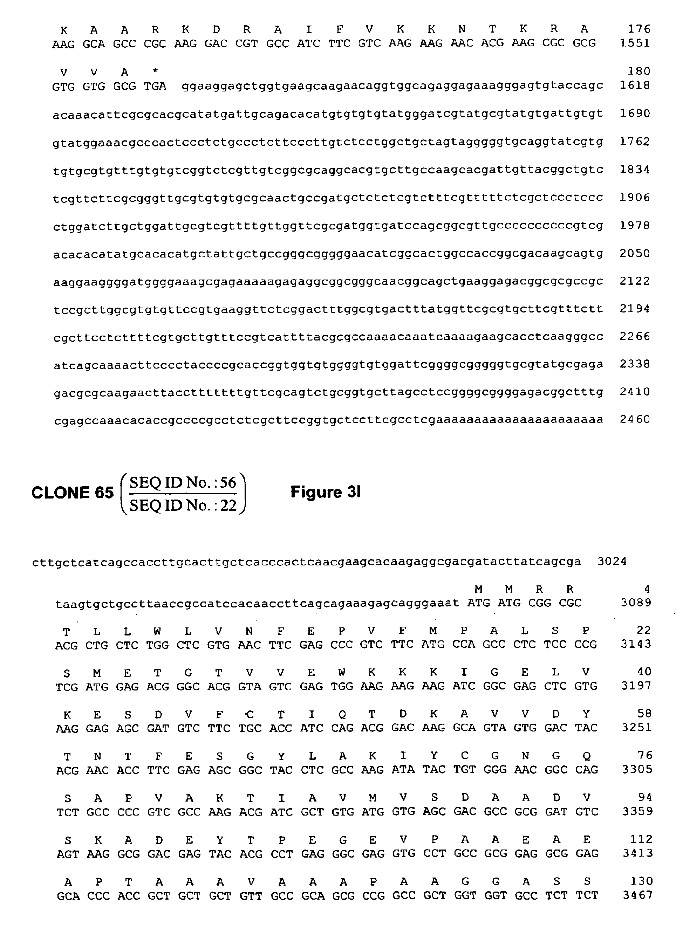

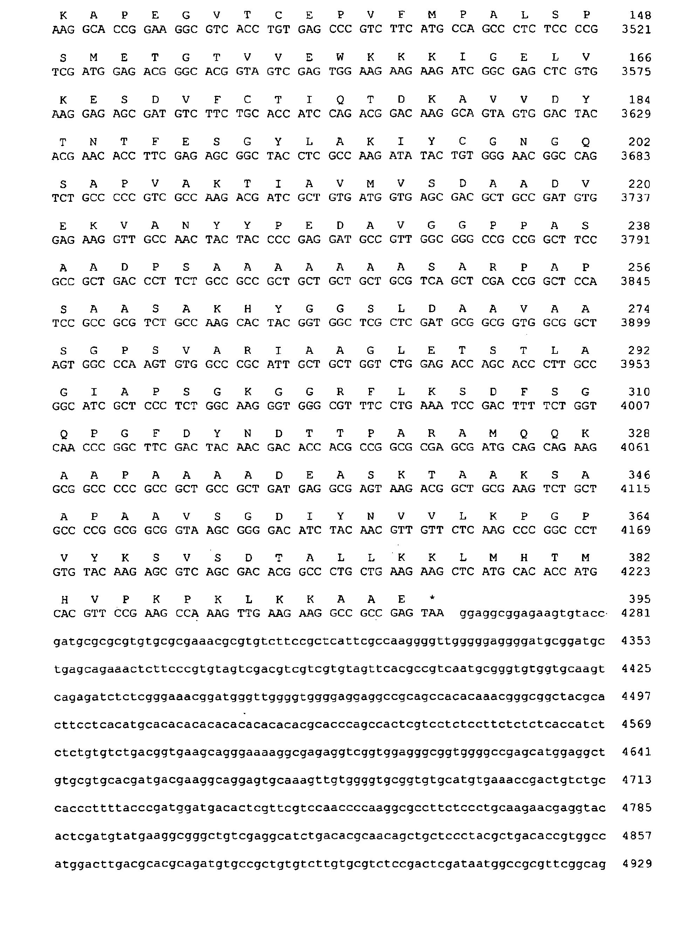

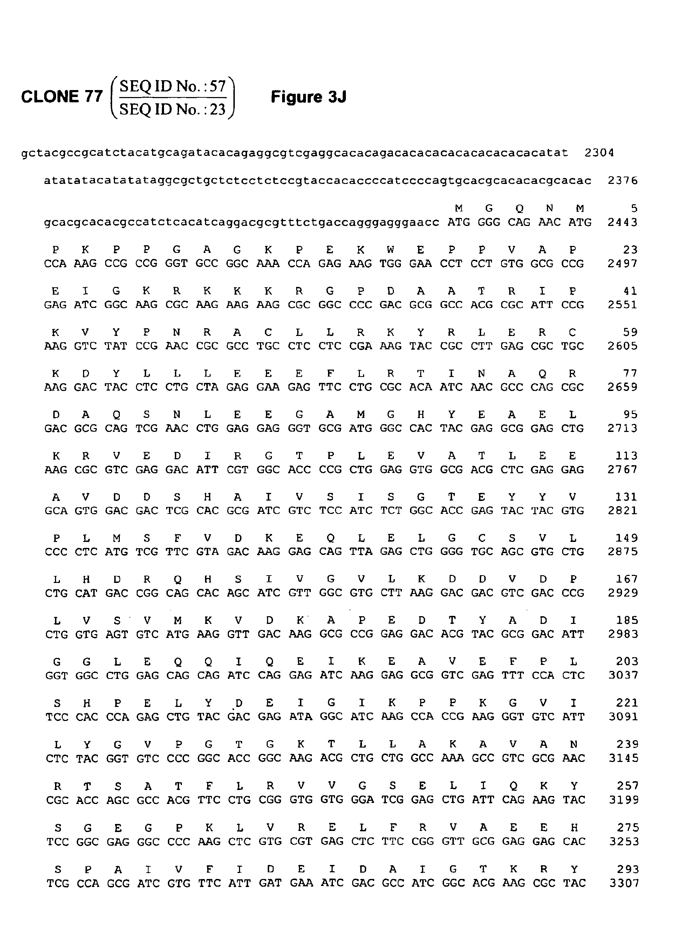

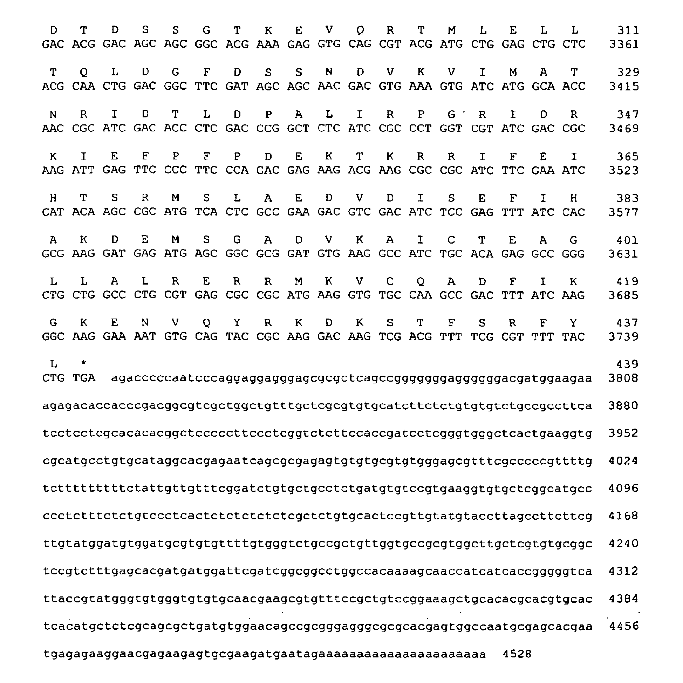

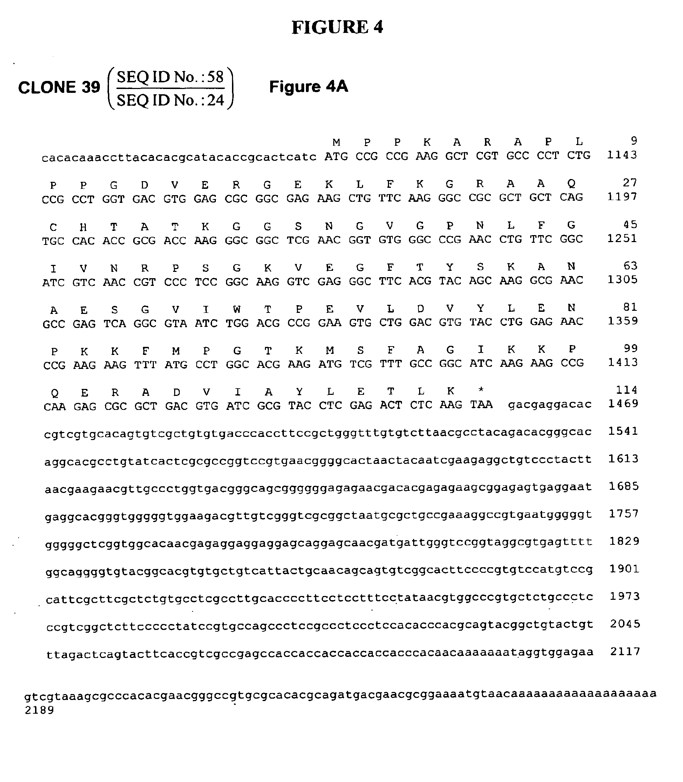

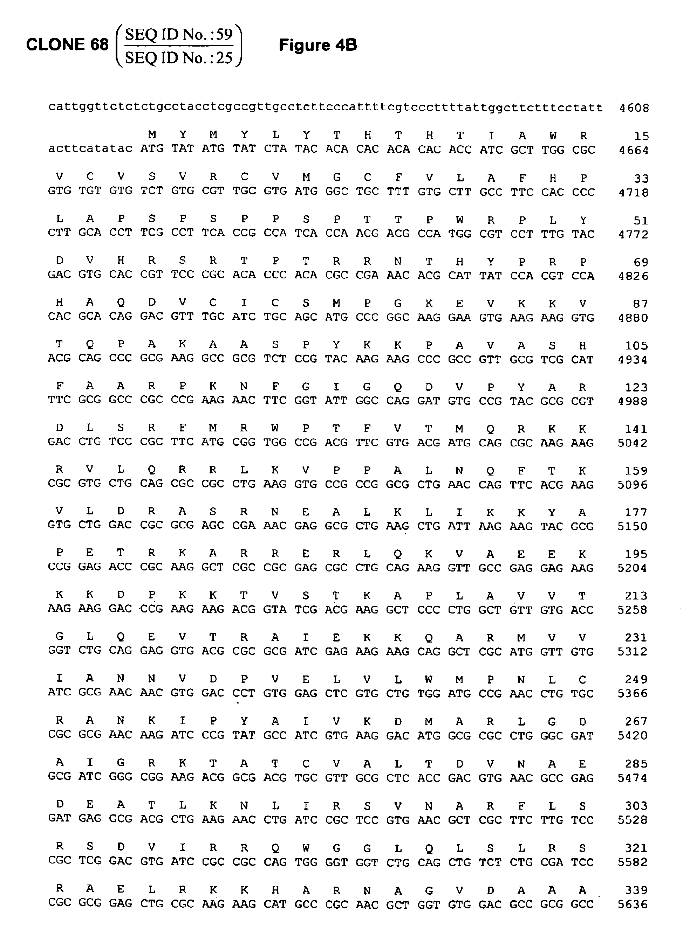

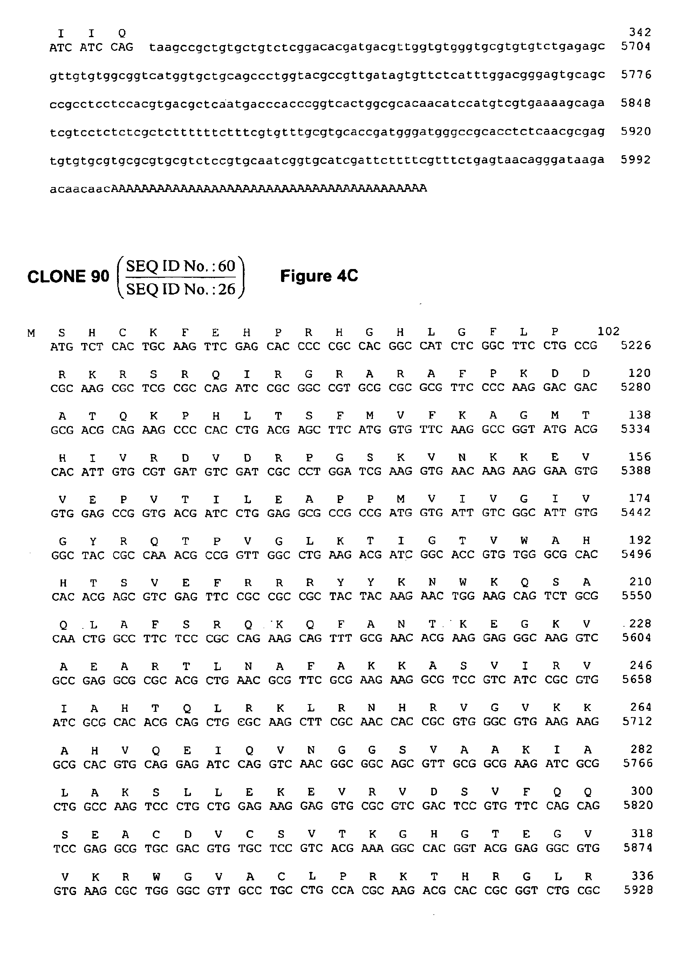

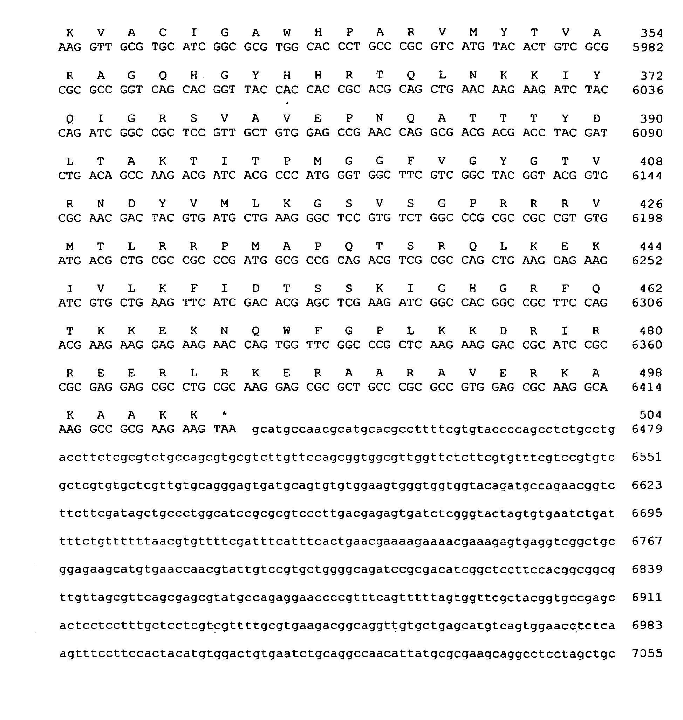

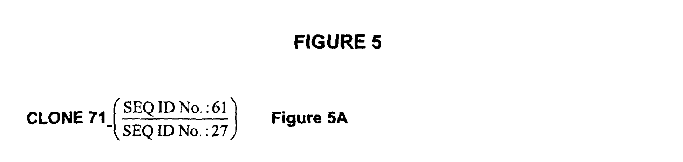

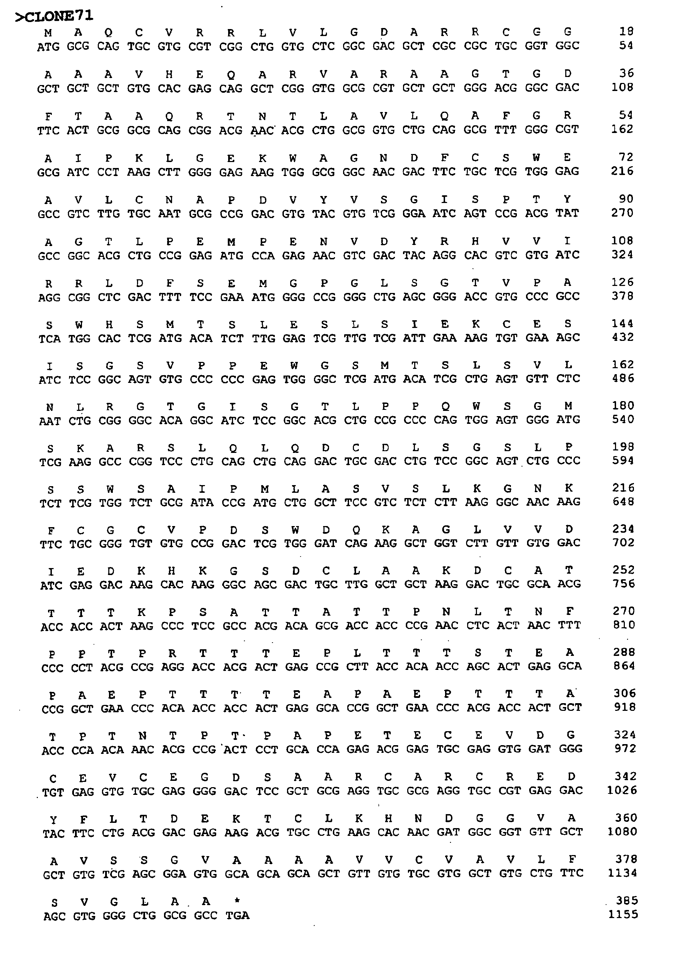

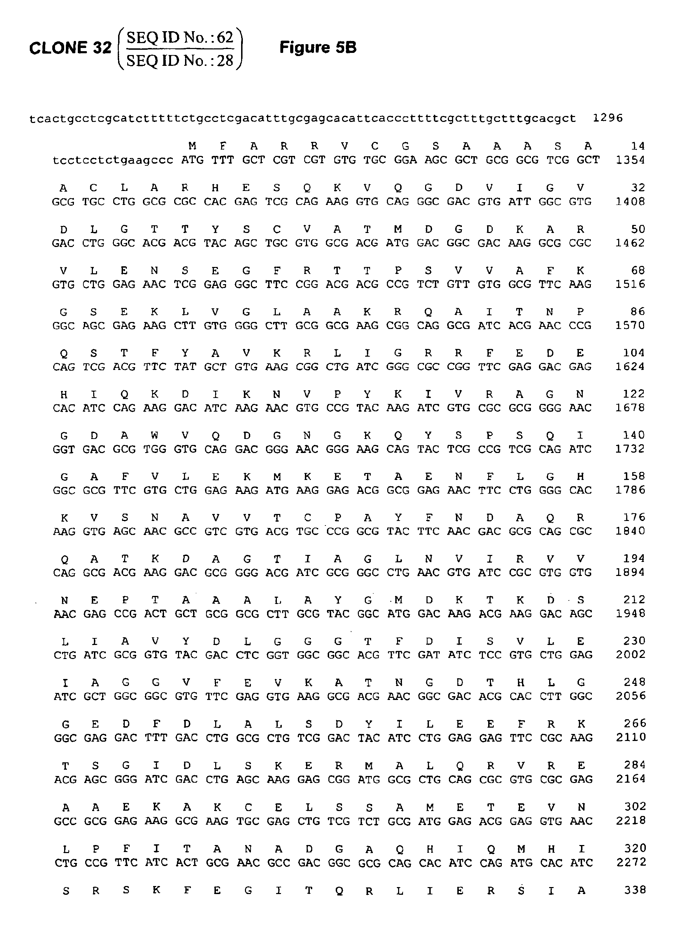

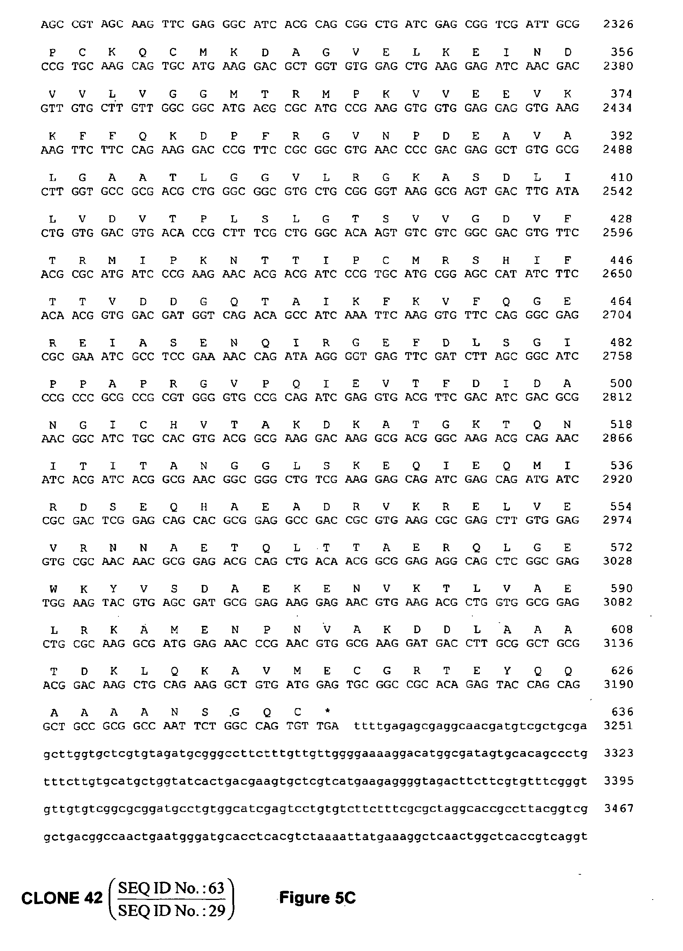

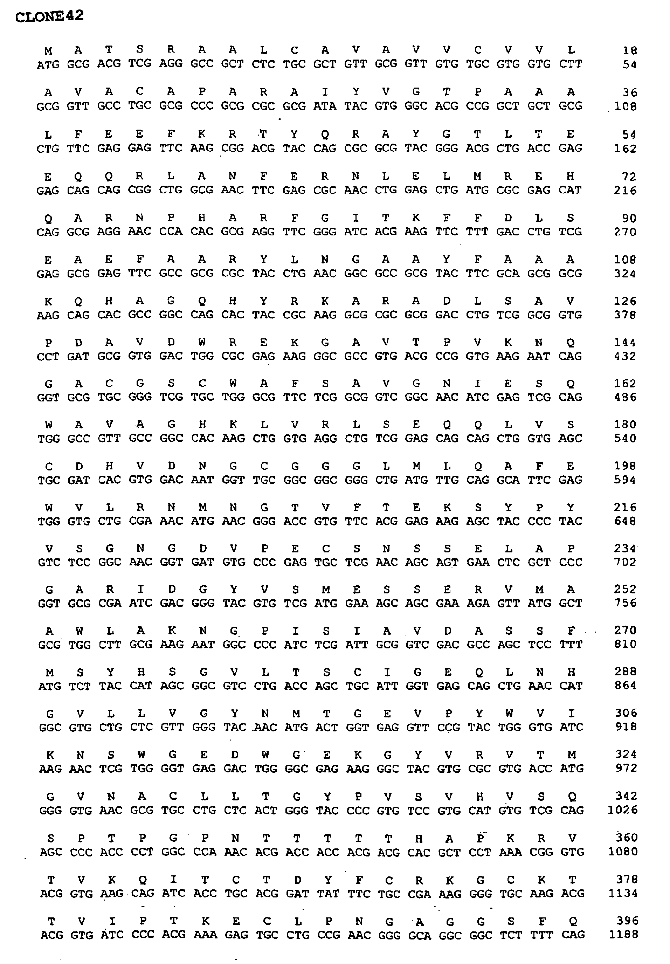

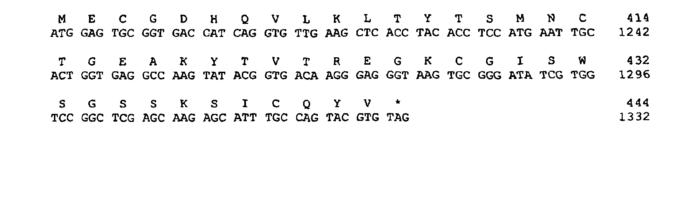

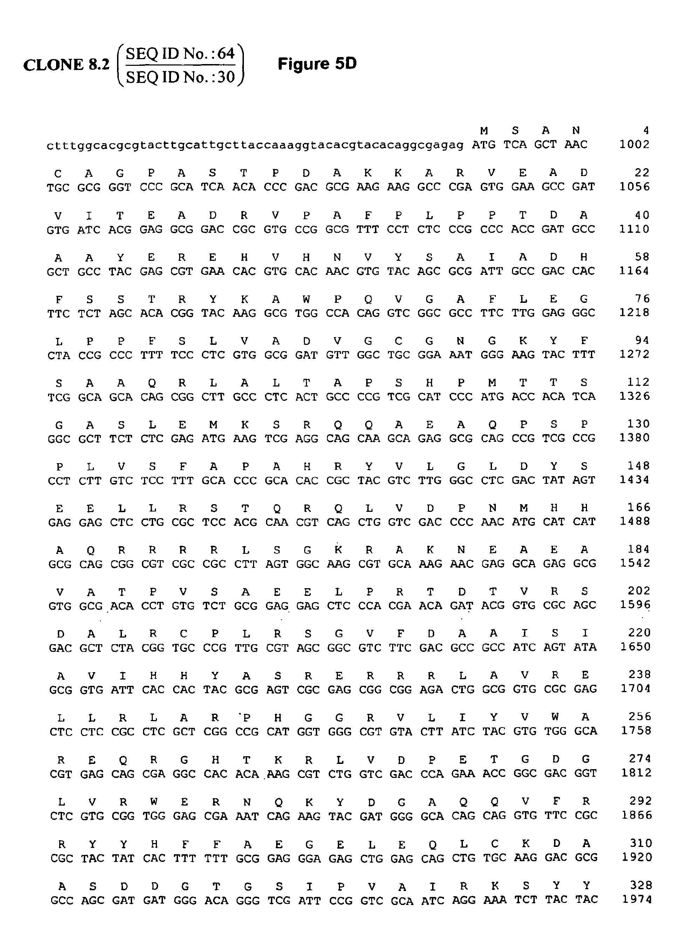

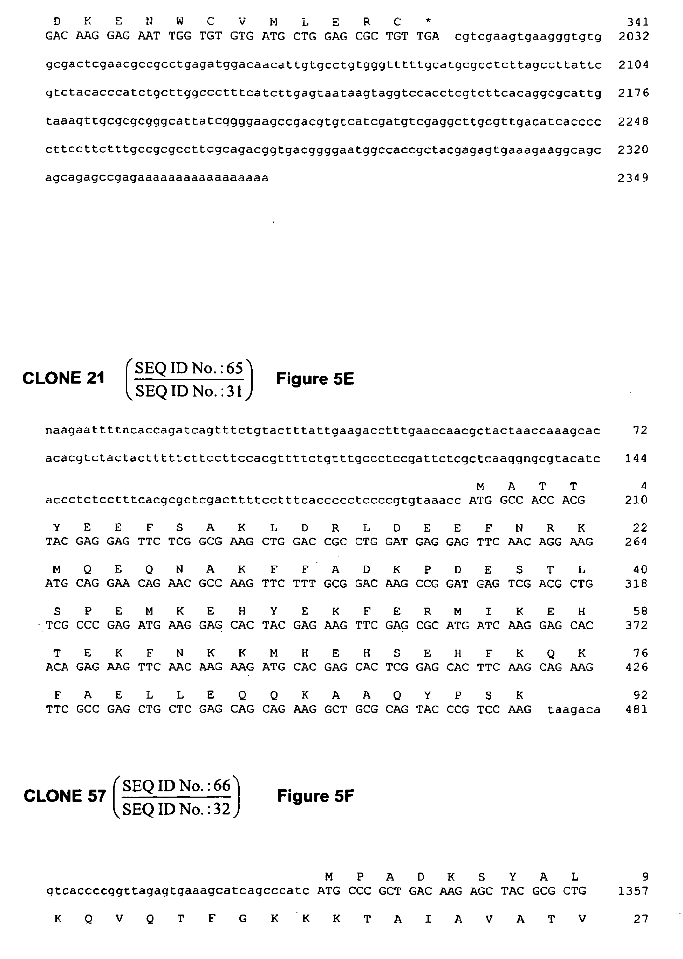

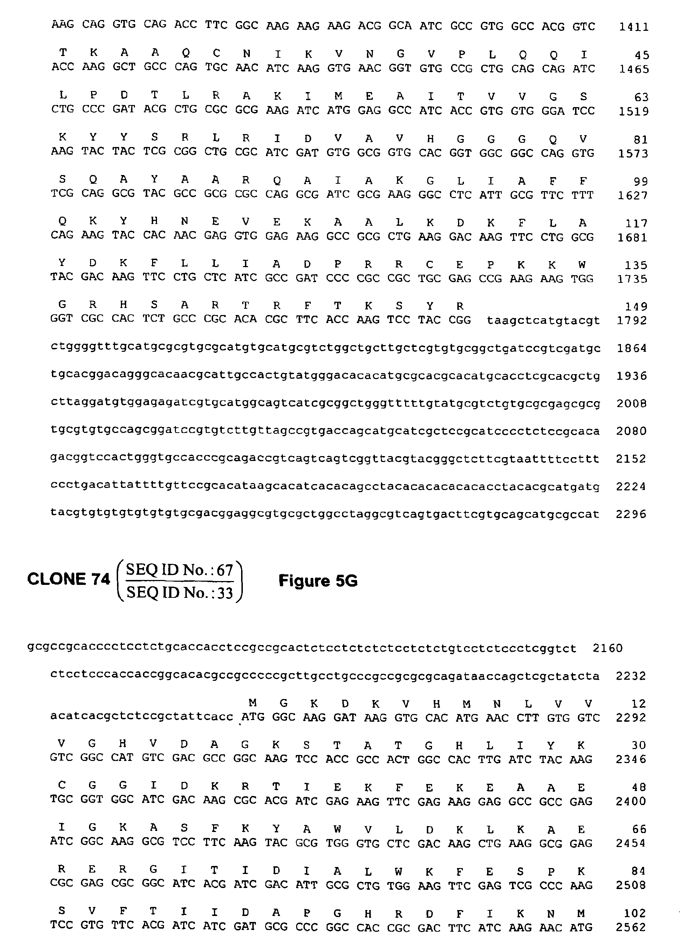

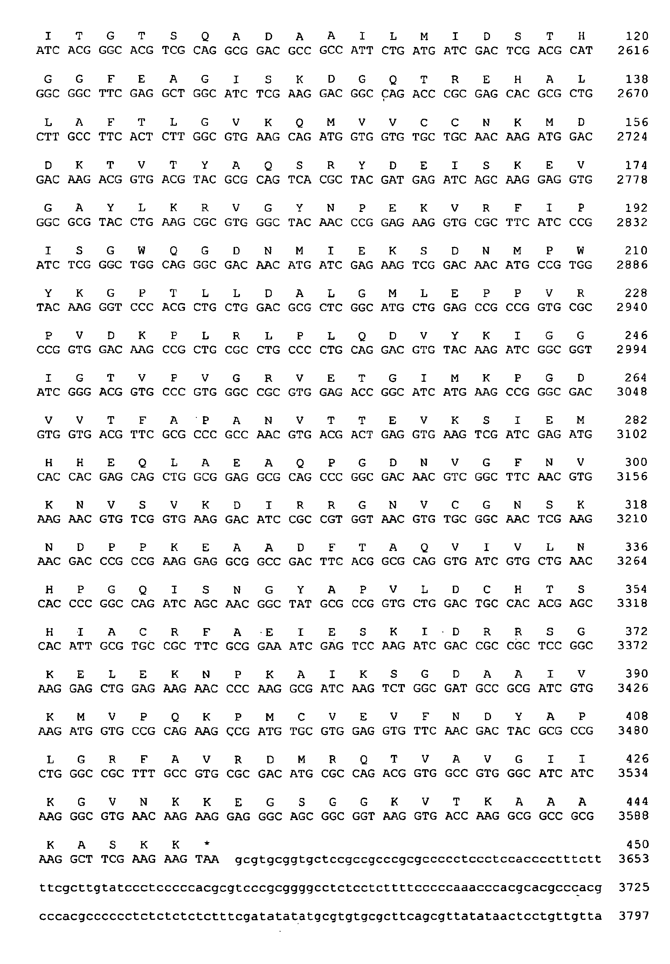

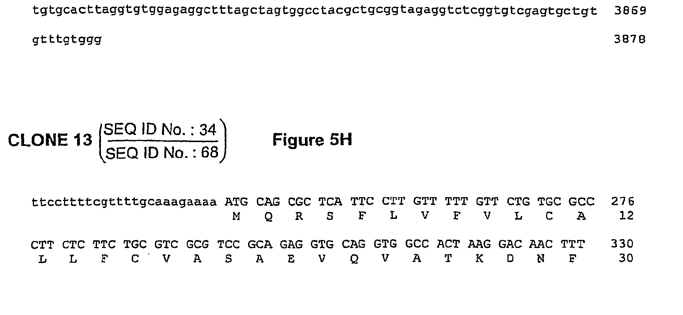

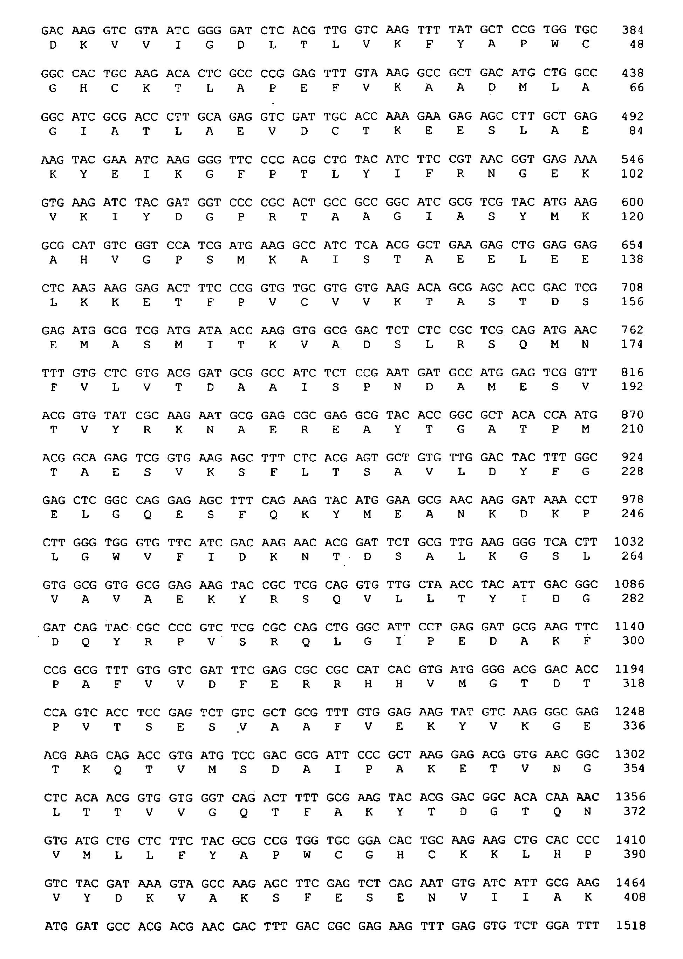

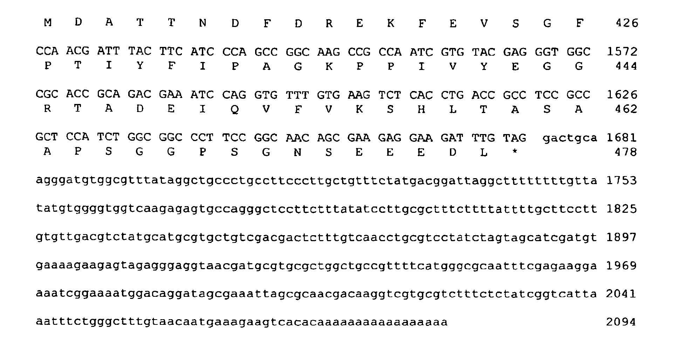

Preferably, the polynucleotide of the application comprises a nucleotide sequence substantially identical to a sequence selected from the group consisting of SEQ ID NOS 1 to 13 (Figure 2; Table 1 : Group 1), or from the group consisting of SEQ ID NOS 14 to 23 (Figure 3; Table 1: Group 2), or from the group consisting of SEQ ID NOS 24 to 26 (Figure 4; Table 1: Group 3) , or from the group consisting of SEQ ID NOS 27 to 34 (Figure 5; Table 1: Group 4).

As used herein, the terms "Isolated or Purified" means altered "by the hand of man" from its natural state, i.e., if it occurs in nature, it has been changed or removed from its original environment, or both. For example, a polynucleotide or a protein/peptide naturally present in a living organism is neither "isolated" nor purified, the same polynucleotide separated from the coexisting materials of its natural state, obtained by cloning, amplification and/or chemical synthesis is "isolated" as the term is employed herein. Moreover, a polynucleotide or a protein/peptide that is introduced into an organism by transformation, genetic manipulation or by any other recombinant method is "isolated" even if it is still present in said organism.

Amino acid or nucleotide sequence "identity" and "similarity" are determined from an optimal global alignment between the two sequences being compared. An optimal global alignment is achieved using, for example, the Needleman-Wunsch algorithm (Needleman and Wunsch, 1970, J. Mol. Biol. 48:443-453). "Identity" means that an amino acid or nucleotide at a particular position in a first polypeptide or polynucleotide is identical to a corresponding amino acid or nucleotide in a second polypeptide or polynucleotide that is in an optimal global alignment with the first polypeptide or polynucleotide . In contrast to identity, "similarity" encompasses amino acids that are conservative substitutions. A "conservative" substitution is any substitution that has a positive score in the blosum62 substitution matrix (Hentikoff and Hentikoff, 1992, Proc. Natl. Acad. Sci. USA 89: 10915-10919). By the statement "sequence A is n% similar to sequence B" is meant that n% of the positions of an optimal global alignment between sequences A and B consists of identical residues or nucleotides and conservative substitutions. By the statement "sequence A is n% identical to sequence B" is meant that n% of the positions of an optimal global alignment between sequences A and B consists of identical residues or nucleotides.

As used herein, the term "polynucleotide(s)" generally refers to any polyribonucleotide or poly-deoxyribonucleotide, which may be unmodified RNA or DNA or modified RNA or DNA. This definition includes, without limitation, single- and double-stranded DNA, DNA that is a mixture of single- and double-stranded regions or single-, double- and triple-stranded regions, single- and double-stranded RNA, and RNA that is mixture of single- and double-stranded regions, hybrid molecules comprising DNA and RNA that may be single-stranded or, more typically, double-stranded, or triple-stranded regions, or a mixture of single- and double-stranded regions. In addition, "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. As used herein, the term "polynucleotide(s)" also includes DNAs or RNAs as described above that contain one or more modified bases. Thus, DNAs or RNAs with backbones modified for stability or for other reasons are "polynucleotide(s)" as that term is intended herein. Moreover, DNAs or RNAs comprising unusual bases, such as inosine, or modified bases, such as tritylated bases, to name just two examples, are polynucleotides as the term is used herein. It will be appreciated that a great variety of modifications have been made to DNA and RNA that serve many useful purposes known to those of skill in the art. "Polynucleotide(s)" embraces short polynucleotides or fragments often referred to as oligonucleotide(s). The term "polynucleotide(s)" as it is employed herein thus embraces such chemically, enzymatically or metabolically modified forms of polynucleotides, as well as the chemical forms of DNA and RNA characteristic of viruses and cells, including, for example, simple and complex cells which exhibits the same biological function as the polypeptide encoded by SEQ ID NO.1 to 34. The term "polynucleotide(s)" also embraces short nucleotides or fragments, often referred to as "oligonucleotides", that due to mutagenesis are not 100% identical but nevertheless code for the same amino acid sequence.

The application also describes an isolated or purified excreted/secreted polypeptide of *Leishmania major* comprising an amino acid sequence substantially identical to a sequence selected from the group consisting of SEQ ID NOS: 35 to 68 and functional derivatives thereof.

In an embodiment, the invention relates to an isolated or purified excreted/secreted polypeptide of *Leishmania major* comprising an amino acid sequence substantially identical to the sequence of SEQ ID NO: 35

By the term "substantially identical", it is meant that the polypeptide of the present invention preferably has an amino sequence having at least 80% homology, or even preferably 85% homology to part or all of SEQ ID NO: 35 to 68.

Yet, more preferably, the polypeptide comprises an amino acid sequence substantially the same or having 100% identity with SEQ ID NO: 35 to 68.

According to a preferred embodiment, the polypeptide of the application invention comprises an amino acid sequence substantially identical to a sequence selected from the group consisting of SEQ ID NOS: 35 to 47 (Annex A; Table 1 : Group 1) or from the group consisting of SEQ ID NOS: 48 to 57 (Annex B; Table 1: Group 2) or from the group consisting of SEQ ID NOS: 58 to 60 (Annex C; Table 1: Group3) or from the group consisting of SEQ ID NOS: 61 to 68 (Annex D; Table 1: Group 4)

A "functional derivative", as is generally understood and used herein, refers to a protein/peptide sequence that possesses a functional biological activity that is substantially similar to the biological activity of the whole protein/peptide sequence. A functional derivative of a protein/peptide may or may not contain post-translational modifications such as covalently linked carbohydrate, if such modification is not necessary for the performance of a specific function. The term "functional derivative" is intended to the "fragments", "segments", "variants", "analogs" or "chemical derivatives" of a protein/peptide.

As used herein, the term "polypeptide(s)" refers to any peptide or protein comprising two or more amino acids joined to each other by peptide bonds or modified peptide bonds. "Polypeptide(s)" refers to both short chains, commonly referred to as peptides, oligopeptides and oligomers and to longer chains generally referred to as proteins. Polypeptides may contain amino acids other than the 20 gene-encoded amino acids. "Polypeptide(s)" include those modified either by natural processes, such as processing and other post-translational modifications, but also by chemical modification techniques. Such modifications are well described in basic texts and in more detailed monographs, as well as in a voluminous research literature, and they are well known to those of skill in the art. It will be appreciated that the same type of modification may be present in the same or varying degree at several sites in a given polypeptide. Also, a given polypeptide may contain many types of modifications. Modifications can occur anywhere in a polypeptide, including the peptide backbone, the amino acid side-chains, and the amino or carboxyl termini. Modifications include, for example, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of cysteine, formation of pyroglutamate, formylation, gamma-carboxylation, GPI anchor formation, hydroxylation, iodination, methylation, myristoylation, oxidation, proteolytic processing, phosphorylation, prenylation, racemization, glycosylation, lipid attachment, sulfation, gamma-carboxylation of glutamic acid residues, hydroxylation, selenoylation, sulfation and transfer-RNA mediated addition of amino acids to proteins, such as arginylation, and ubiquitination. See, for instance: PROTEINS-STRUCTURE AND MOLECULAR PROPERTIES, 2nd Ed., T. E. Creighton, W.H. Freeman and Company, New York (1993); Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York (1983); Seifter et al., Meth. Enzymol. 182:626-646 (1990); and Rattan et al., Protein Synthesis: Posttranslational Modifications and Aging, Ann. N.Y. Acad. Sci. 663: 48-62(1992). Polypeptides may be branched or cyclic, with or without branching. Cyclic, branched and branched circular polypeptides may result from post-translational natural processes and may be made by entirely synthetic methods, as well.

### 2. Vectors and Cells

The application describes and the invention is also directed to a host, such as a genetically modified cell, comprising any of the polynucleotide sequence according to the application or of the invention, respectively and more preferably, a host capable of expressing the polypeptide encoded by this polynucleotide.

Transformed or transfected cells preferably contemplated by the application contain a polynucleotide having a sequence comprising a nucleotide sequence substantially identical to a sequence selected from the group consisting of SEQ ID NOS 1 to 13 and functional fragments thereof. Examples of such cells are those consisting of an *Escherichia coli* bacterium selected from the group consisting of *Escherichia coli* bacteria filed at the CNCM. under accession numbers 1-3394, I-3393, I-3395, I-3396, I-3377, I-3371, I-3376, I-3373, I-3379, I-3397, I-3384, I-3383 and I-3382 on February 24 2005.

Transformed or transfected cells preferably contemplated by the invention contain a polynucleotide having a sequence comprising a nucleotide sequence substantially identical to the sequence of SEQ ID NO: 1 and functional fragments thereof. Examples of such cells are those consisting of an *Escherichia coli* bacterium selected from the group consisting of *Escherichia coli* bacteria filed at the CNCM under accession number I-3394.

Other transformed or transfected cells preferably contemplated by the application contain a polynucleotide having a sequence comprising a nucleotide sequence substantially identical to a sequence selected from the group consisting of SEQ ID NOS 14 to 23 and functional fragments thereof. Examples of such cells are those consisting of an *Escherichia coli* bacterium selected from the group consisting of *Escherichia coli* bacteria filed at the CNCM. under accession numbers I-3386, I-3378, I-3385, I-3381, I-3372, I-3392, I-3380, I-3367, I-3370, and I-3366 on February 24, 2005.

Other transformed or transfected cells preferably contemplated by the application contain a polynucleotide having a sequence comprising a nucleotide sequence substantially identical to a sequence selected from the group consisting of SEQ ID NOS 24 to 26 and functional fragments thereof. Examples of such cells are those consisting of an *Escherichia coli* bacterium selected from the group consisting of *Escherichia coli* bacteria filed at the CNCM. under accession numbers I-3365, I-3369 and I-3368 on February 24, 2005.

Other transformed or transfected cells preferably contemplated by the application contain a polynucleotide having a sequence comprising a nucleotide sequence substantially identical to a sequence selected from the group consisting of SEQ ID NOS 27 to 34 and functional fragments thereof. Examples of such cells are those consisting of an *Escherichia coli* bacterium selected from the group consisting of *Escherichia coli* bacteria filed at the CNCM. under accession numbers I-3364, I-3387, I-3391, I-3389, I-3390, I-3388, I-3374, and I-3375 on February 24, 2005.

The application describes and the invention is further directed to cloning or expression vector comprising a polynucleotide sequence as respectively defined above, and more particularly directed to a cloning or expression vector which is capable of directing expression of the polypeptide encoded by the polynucleotide sequence in a vector-containing cell.

As used herein, the term "vector" refers to a polynucleotide construct designed for transduction/transfection of one or more cell types. Vectors may be, for example, "cloning vectors" which are designed for isolation, propagation and replication of inserted nucleotides, "expression vectors" which are designed for expression of a nucleotide sequence in a host cell, or a "viral vector" which is designed to result in the production of a recombinant virus or virus-like particle, or "shuttle vectors", which comprise the attributes of more than one type of vector.

A number of vectors suitable for stable transfection of cells and bacteria are available to the public (e.g. plasmids, adenoviruses, baculoviruses, yeast baculoviruses, plant viruses, adeno-associated viruses, retroviruses, Herpes Simplex Viruses, Alphaviruses, Lentiviruses), as are methods for constructing such cell lines. It will be understood that the present invention encompasses any type of vector comprising any of the polynucleotide molecule of the invention.

The application describes genetically modified Leishmania strains. A first preferred genetically modified Leishmania strain comprises at least one gene having a sequence comprising a nucleotide sequence substantially identical to a sequence selected from the group consisting of SEQ ID NOS 1 to 34, and wherein said at least one gene is inactivated, preferably by knock-out. A second preferred genetically modified Leishmannia strain contemplated by the present invention comprises at least one gene having a sequence comprising a nucleotide sequence substantially identical to a sequence selected from the group consisting of SEQ ID NOS 1 to 34, and wherein said at least one gene is underexpressed compared to a corresponding gene of a wild-type strain of Leishmania. The invention relates to genetically modified Leishmania strains, which comprise at least one gene having a sequence comprising a nucleotide sequence substantially identical to the sequence of SEQ ID NO: 1, and wherein said at least one gene is inactivated, preferably by knock-out, or wherein said at least one gene is underexpressed compared to a corresponding gene of a wild-type strain of Leishmania. Methods by which such strains are genetically modified are known to one skilled in the art and will not be further discussed.

### 3. Antibodies

The application describes and the invention relates to purified antibodies that specifically bind to the isolated or purified polypeptide of the application or of the invention as respectively defined above or fragments thereof. The antibodies of the application and of the invention may be prepared by a variety of methods using the polypeptides described above. For example, the polypeptide, or antigenic fragments thereof, may be administered to an animal in order to induce the production of polyclonal antibodies. Alternatively, antibodies used as described herein may be monoclonal antibodies, which are prepared using hybridoma technology (see, e.g., Hammering et al., In Monoclonal Antibodies and T-Cell Hybridomas, Elsevier, NY, 1981).

As mentioned above, the application describes and the invention is preferably directed to antibodies that specifically bind to *Leishmanina major* excreted/secreted polypeptides, or fragments thereof as defined above. In particular, the invention features "neutralizing" antibodies. By "neutralizing" antibodies is meant antibodies that interfere with any of the biological activities of any of the *Leishmanina major* excreted/secreted polypeptides. Any standard assay known to one skilled in the art may be used to assess potentially neutralizing antibodies. Once produced, monoclonal and polyclonal antibodies are preferably tested for specific *Leishmanina major* excreted/secreted polypeptides recognition by Western blot, immunoprecipitation analysis or any other suitable method.

With respect to the antibodies of the application and to the antibodies of the invention, the term "specifically binds to" refers to antibodies that bind with a relatively high affinity to one or more epitopes of a protein of interest, but which do not substantially recognize and bind molecules other than the one(s) of interest. As used herein, the term "relatively high affinity" means a binding affinity between the antibody and the protein of interest of at least 10⁶ M⁻¹, and preferably of at least about 10⁷ M⁻¹ and even more preferably 10⁸ M⁻¹ to 10¹⁰ M⁻¹. Determination of such affinity is preferably conducted under standard competitive binding immunoassay conditions which is common knowledge to one skilled in the art. As used herein, "antibody" and "antibodies" include all of the possibilities mentioned hereinafter: antibodies or fragments thereof obtained by purification, proteolytic treatment or by genetic engineering, artificial constructs comprising antibodies or fragments thereof and artificial constructs designed to mimic the binding of antibodies or fragments thereof. Such antibodies are discussed in Colcher et al. (Q J Nucl Med 1998; 42: 225-241). They include complete antibodies, F(ab')₂ fragments, Fab fragments, Fv fragments, scFv fragments, other fragments, CDR peptides and mimetics. These can easily be obtained and prepared by those skilled in the art. For example, enzyme digestion can be used to obtain F(ab')₂ and Fab fragments by subjecting an IgG molecule to pepsin or papain cleavage respectively. Recombinant antibodies are also covered by the present invention.

Preferably, the antibody of the application or of the invention is a human or animal immunoglobulin such as IgG1, IgG2, IgG3, IgG4, IgM, IgA, IgE or IgD carrying rat or mouse variable regions (chimeric) or CDRs (humanized or "animalized"). Furthermore, the antibody of the application or of the invention may also be conjugated to any suitable carrier known to one skilled in the art in order to provide, for instance, a specific delivery and prolonged retention of the antibody, either in a targeted local area or for a systemic application.

The term "humanized antibody" refers to an antibody derived from a non-human antibody, typically murine, that retains or substantially retains the antigen-binding properties of the parent antibody but which is less immunogenic in humans. This may be achieved by various methods including (a) grafting only the non-human CDRs onto human framework and constant regions with or without retention of critical framework residues, or (b) transplanting the entire non-human variable domains, but "cloaking" them with a human-like section by replacement of surface residues. Such methods are well known to one skilled in the art.

As mentioned above, the antibody of the application or of the invention is immunologically specific to the polypeptide of the application or of the invention, respectively, and immunological derivatives thereof. As used herein, the term "immunological derivative" refers to a polypeptide that possesses an immunological activity that is substantially similar to the immunological activity of the whole polypeptide, and such immunological activity refers to the capacity of stimulating the production of antibodies immunologically specific to the *Leishmanina major* excreted/secreted polypeptides or derivative thereof. The term "immunological derivative" therefore encompass "fragments", "segments", "variants", or "analogs" of a polypeptide.

### 4. Compositions and vaccines

The polypeptides of the application and of the invention, the polynucleotides coding the same, and antibodies produced according to the application or to the invention, may be used in many ways for the diagnosis, the treatment or the prevention of Leishmaniasis.

The application describes an immunogenic composition generating an immune response against a leishmaniasis, comprising a polynucleotide of the application as defined above or a polypeptide of the application as defined above, and an acceptable carrier. According to a related aspect, the application describes a vaccine composition generating a protecting response against a leishmaniasis, comprising a polynucleotide of the application as defined above or a polypeptide of the application as defined above, and an acceptable carrier.

In an embodiment, the invention relates to an immunogenic composition generating an immune response against a leishmaniasis, comprising a polynucleotide of the invention as defined above or a polypeptide of the invention as defined above, and an acceptable carrier. According to a related aspect, the invention relates to a vaccine composition generating a protecting response against a leishmaniasis, comprising a polynucleotide of the invention as defined above or a polypeptide of the invention as defined above, and an acceptable carrier.

As used herein, the term "treating" refers to a process by which the symptoms of Leishmaniasis are alleviated or completely eliminated. As used herein, the term "preventing" refers to a process by which a Leishmaniasis is obstructed or delayed. The composition of the vaccine of the application or of the invention comprises a polynucleotide and/or a polypeptide as defined above and an acceptable carrier.

As used herein, the expression "an acceptable carrier" means a vehicle for containing the polynucleotide and/or a polypeptide that can be injected into a mammalian host without adverse effects. Suitable carriers known in the art include, but are not limited to, gold particles, sterile water, saline, glucose, dextrose, or buffered solutions. Carriers may include auxiliary agents including, but not limited to, diluents, stabilizers (i. e., sugars and amino acids), preservatives, wetting agents, emulsifying agents, pH buffering agents, viscosity enhancing additives, colors and the like.

Further agents can be added to the composition and vaccine of the application or of the invention. For instance, the composition of the invention may also comprise agents such as drugs, immunostimulants (such as α-interferon, β-interferon, γ-interferon, granulocyte macrophage colony stimulator factor (GM-CSF), macrophage colony stimulator factor (M-CSF), interleukin 2 (IL2), interleukin 12 (IL12), and CpG oligonucleotides), antioxidants, surfactants, flavoring agents, volatile oils, buffering agents, dispersants, propellants, and preservatives. For preparing such compositions, methods well known in the art may be used.

The amount of polynucleotide and/or a polypeptide present in the compositions of the application and in the compositions of the invention is preferably a therapeutically effective amount. A therapeutically effective amount of polynucleotide and/or a polypeptide is that amount necessary to allow the same to perform their immunological role without causing, overly negative effects in the host to which the composition is administered. The exact amount of polynucleotide and/or a polypeptide to be used and the composition/vaccine to be administered will vary according to factors such as the type of condition being treated, the mode of administration, as well as the other ingredients in the composition.

### 5. Method for identifying a polypeptide of the invention

In another object, the invention provides a method for identifying an excreted/secreted polypeptide of a *Leishmania major* strain. The method comprises *in vitro* cultivating Leishmania promastigotes under pH and temperature conditions naturally found in a host cell infected by a *Leishmania major* strain. Preferably, the pH is about 5.5 and the temperature is about 35°C. By "about", it is meant that the value of said pH or temperature can vary within a certain range depending on the margin of error of the method used to evaluate such pH or temperature.

In a related aspect, the excreted/secreted polypeptides identified by the method as defined above finds a particular use as drug target for identifying a molecule capable of preventing a Leishmaniasis.

### 6. Methods of use

The application describes and the invention relates to a method for preventing and/or treating a patient against an infection with a *Leishmania major* strain, the method comprising the step of administering to the patient a therapeutically effective amount of a immunogenic and/or a vaccine composition of the application or of the invention as respectively defined above and/or an antibody of the application or of the invention as respectively as defined above.

The vaccine, antibody and immunogenic composition of the application or of the invention may be given to a patient through various routes of administration. For instance, the composition may be administered in the form of sterile injectable preparations, such as sterile injectable aqueous or oleaginous suspensions. These suspensions may be formulated according to techniques known in the art using suitable dispersing or wetting agents and suspending agents. The sterile injectable preparations may also be sterile injectable solutions or suspensions in non-toxic parenterally-acceptable diluents or solvents. They may be given parenterally, for example intravenously, intramuscularly or subcutaneously by injection, by infusion or *per os.* The vaccine and the composition of the invention may also be formulated as creams, ointments, lotions, gels, drops, suppositories, sprays, liquids or powders for topical administration. They may also be administered into the airways of a subject by way of a pressurized aerosol dispenser, a nasal sprayer, a nebulizer, a metered dose inhaler, a dry powder inhaler, or a capsule. Suitable dosages will vary, depending upon factors such as the amount of each of the components in the composition, the desired effect (short or long term), the route of administration, the age and the weight of the mammal to be treated. Any other methods well known in the art may be used for administering the vaccine, antibody and the composition of the application or of the invention.

The application describes and the invention is also directed to an *in vitro* diagnostic method for the detection of the presence or absence of antibodies indicative of a *Leishmania major* strain, which bind to a polypeptide of the application or the invention as respectively defined above to form an immune complex, comprising the steps of
a) contacting said polypeptide with a biological sample for a time and under conditions sufficient to form an immune complex; and
b) detecting the presence or absence of the immune complex formed in a).

The application describes and the invention relates to a diagnostic kit for the detection of the presence or absence of antibodies indicative of of a *Leishmania major* strain. Accordingly, the kit comprises:
- a polypeptide of the application or of the invention as respectively defined above;
- a reagent to detect polypeptide-antibody immune complex;
- optionally a biological reference sample lacking antibodies that immunologically bind with the polypeptide; and
- optionally a comparison sample comprising antibodies which can specifically bind to the polypeptide;
wherein the polypeptide, reagent, biological reference sample, and comparison sample are present in an amount sufficient to perform the detection.

The application describes and the invention relates to an *in vitro* diagnostic method for the detection of the presence or absence of polypeptides indicative a *Leishmania major* strain, which bind to the antibody of the application or of the invention, respectively, to form an immune complex, comprising the steps of:
a) contacting said antibody with a biological sample for a time and under conditions sufficient to form an immune complex; and
b) detecting the presence or absence of the immune complex formed in a).

The application describes and the invention relates to a diagnostic kit for the detection of the presence or absence of polypeptides indicative of *Leishmania major* strain. Accordingly, the kit comprises:
- an antibody of the application or of the invention as respectively defined above;
- a reagent to detect polypeptide-antibody immune complex;
- optionally a biological reference sample lacking polypeptides that immunologically bind with the antibody; and
- optionally a comparison sample comprising polypeptides which can specifically bind to the antibody;
wherein said antibody, reagent, biological reference sample, and comparison sample are present in an amount sufficient to perform the detection.

A "biological sample" encompasses a variety of sample types obtained from an individual and can be used in a diagnostic or monitoring assay. The definition encompasses blood and other liquid samples of biological origin, solid tissue samples such as a biopsy specimen or tissue cultures or cells derived therefrom, and the progeny thereof. The definition also includes samples that have been manipulated in any way after their procurement, such as by treatment with reagents, solubilization, or enrichment for certain components, such as proteins or polynucleotides. The term "biological sample" encompasses a clinical sample, and also includes cells in culture, cell supernatants, cell lysates, serum, plasma, biological fluid, and tissue samples.

The application describes and the invention relates to a method for detecting the presence or absence of lymphocytic stimulation in a subject suspected of Leishmaniasis, comprising the steps of:
a) obtaining a sample containing T Lymphocytes from said subject;
b) contacting the T lymphocytes with a polypeptide of the application or of the invention, respectively; and
c) detecting the presence or absence of a proliferative response of said T lymphocyte to the polypeptide.

The application describes and the invention relates to a method for detecting the presence or absence of lymphocytic stimulation in a subject suspected of Leishmaniasis, comprising the steps of:
a) obtaining a sample containing T Lymphocytes from said subject;
b) contacting the T lymphocytes with a polypeptide of the application or of the invention, respectively; and
c) detecting the presence or absence of cytokines indicative of lymphocytic stimulation.

The present invention will be more readily understood by referring to the following example. This example is illustrative of the wide range of applicability of the present invention and is not intended to limit its scope. Modifications and variations can be made. Although any method and material similar or equivalent to those described herein can be used in the practice for testing of the present invention, the preferred methods and materials are described.

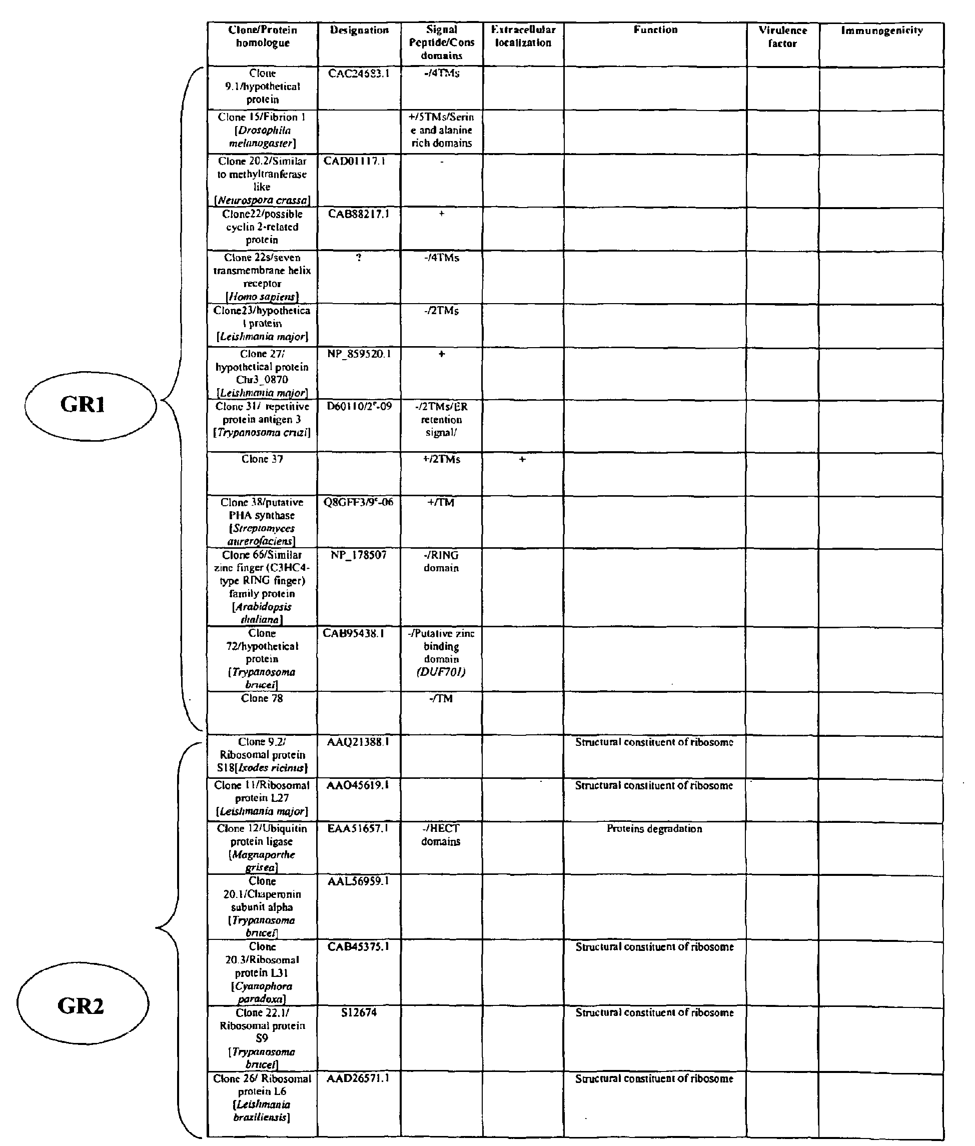

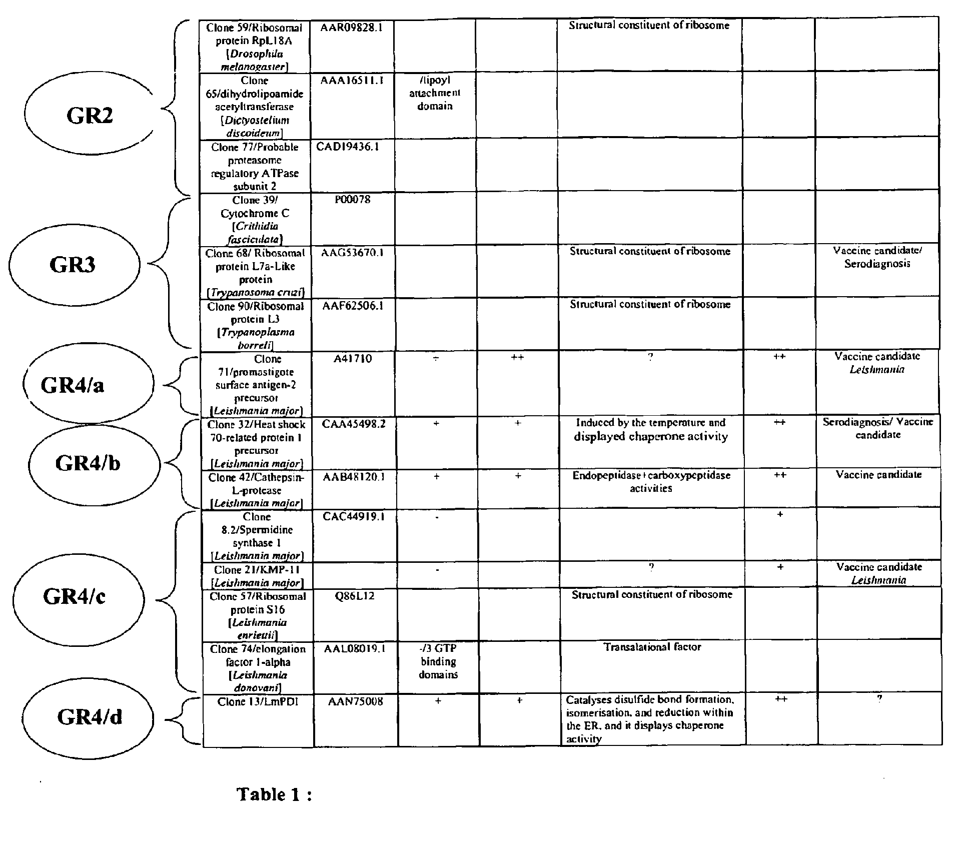

**Table 2**

| **I number CNCM (Paris France)** | **SEQ ID Number** | **Name of the inserted plasmid.** |
|---|---|---|
| I-3394 | ID 1 | pMOS-9.1 |
| I-3393 | ID 2 | pBK-15 |
| I-3395 | ID 3 | pMOS-20.2 |
| I-3396 | ID 4 | pBK-22 |
| I-3377 | ID 5 | pBK-22s |
| I-3371 | ID 6 | pBK-23 |
| I-3376 | ID 7 | pBK-27 |
| I-3373 | ID 8 | pBK-31 |
| I-3379 | ID 9 | pBK-37 |
| I-3397 | ID 10 | pBK-38 |
| I-3384 | ID 11 | pBK-66 |
| I-3383 | ID 12 | pBK-72 |
| I-3382 | ID 13 | pBK-78 |

**Table 3**

| **I number CNCM (Paris France)** | **SEQ ID Number** | **Name of the inserted plasmid.** |
|---|---|---|
| I-3386 | ID 14 | pMOS-9.2 |
| I-3378 | ID 15 | pBK-11 |
| I-3385 | ID 16 | pBK-12 |
| I-3381 | ID 17 | pBK-20.1 |
| I-3372 | ID 18 | pBK-20.3 |
| I-3392 | ID 19 | pMOS-22.1 |
| I-3380 | ID 20 | pBK-26 |
| I-3367 | ID 21 | pBK-59 |
| I-3370 | ID 22 | pBK-65 |
| I-3366 | ID 23 | pBK-77 |

**Table 4**

| **I number CNCM (Paris France)** | **SEQ ID Number** | **Name of the inserted plasmid.** |
|---|---|---|
| I-3365 | ID 24 | pBK-39 |
| I-3369 | ID 25 | pBK-68 |
| I-3368 | ID 26 | pBK-90 |

**Table 5**

| **I number CNCM (Paris France)** | **SEQ ID Number** | **Name of the inserted plasmid.** |
|---|---|---|
| I-3364 | ID 27 | pBK-71 |
| I-3387 | ID 28 | pBK-32 |
| I-3391 | ID 29 | pBK-42 |
| I-3389 | ID 30 | pMOS-8.2 |
| I-3390 | ID 31 | pBK-21 |
| I-3388 | ID 32 | pBK-57 |
| I-3374 | ID 33 | pBK-74 |
| I-3375 | ID 34 | pBK-13 |

### Description of characterizing features for the deposited biological material

pBK15: E. *coli XL1-Blue* bacteria are transformed by the pBK-CMV plasmid (Amersham) containing the DNA sequence coding for protein 15 of *Leishmania major.* Gene 15 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pMOS-20.2: *E. coli XL1-Blue* bacteria are transformed by the pMOS-Blue plasmid (Amersham) containing the DNA sequence coding for protein 20.2 of *Leishmania major.* Gene 20.2 has been cloned at the *EcoR* V restriction site. The recombinant (transformed) bacteria are resistant to tetracycline and ampicillin. The genes which give resistance to ampicillin and to tetracycline are carried by the recombinant pMOS-Blue plasmid and XL1-Blue bacteria, respectively.

pBK22: *E. coli XL1-Blue* bacteria are transformed by the pBK-CMV plasmid (Amersham) containing the DNA sequence coding for protein 22 of *Leishmania major.* Gene 22 has been cloned at the *Xhol* and EcoR I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-22s: *E. coli XL1-Blue* bacteria are transformed by the pBK-CMV plasmid (Amersham) containing the DNA sequence coding for protein 22s of *Leishmania major.* Gene 22s has been cloned at the *Xhol* and EcoR I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-23: *E. coli XL1-Blue* bacteria are transformed by the pBK-CMV plasmid (Amersham) containing the DNA sequence coding for protein 23 of *Leishmania major.* Gene 23 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-27: *E. coli XL1-Blue* bacteria are transformed by the pBK-CMV plasmid (Amersham) containing the DNA sequence coding for protein 27 of *Leishmania major.* Gene 27 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-31: *E. coli XL1-Blue* bacteria are transformed by the pBK-CMV plasmid (Amersham) containing the DNA sequence coding for protein 31 of *Leishmania major.* Gene 31 has been cloned at the *Xhol* and EcoR I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-37: *E. coli XL1-Blue* bacteria are transformed by the pBK-CMV plasmid (Amersham) containing the DNA sequence coding for protein 37 of *Leishmania major.* Gene 37 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

Bp K-38: *E. coli XL1-Blue* bacteria are transformed by the pBK-CMV plasmid (Amersham) containing the DNA sequence coding for protein 38 of *Leishmania major.* Gene 38 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-66: *E. coli XL1-Blue* bacteria are transformed by the pBK-CMV plasmid (Amersham) containing the DNA sequence coding for protein 66 of *Leishmania major.* Gene 66 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-72: *E. coli XL1-Blue* bacteria are transformed by the pBK-CMV plasmid (Amersham) containing the DNA sequence coding for protein 72 of *Leishmania major.* Gene 72 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-78: *E. coli XL1-Blue* bacteria are transformed by the pBK-CMV plasmid (Amersham) containing the DNA sequence coding for protein 78 of *Leishmania major.* Gene 78 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pMOS-9.2: E. *coli XL1-Blue* bacteria are transformed by the pMOS-Blue plasmid (Amersham) containing the DNA sequence coding for protein 9.2 of *Leishmania major.* Gene 9.2 has been cloned at the EcoR V restriction site. The recombinant (transformed) bacteria are resistant to tetracycline and ampicillin. The genes which give resistance to ampicillin and to tetracycline are carried by the recombinant pMOS-Blue plasmid and XL1-Blue bacteria, respectively.

pBK-11: *E. coli XL1-Blue* bacteria are transformed by the pBK-CMV plasmid (Amersham) containing the DNA sequence coding for protein 11 of *Leishmania major.* Gene 11 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-12: *E. coli XL1-Blue* bacteria are transformed by the pBK-CMV plasmid (Amersham) containing the DNA sequence coding for protein 12 of *Leishmania major.* Gene 12 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-20.1: *E. coli XL1-Blue* bacteria are transformed by the pBK-CMV plasmid (Amersham) containing the DNA sequence coding for protein 20.1 of *Leishmania major.* Gene 20.1 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-20.3: *E. coli XL1-Blue* bacteria are transformed by the pBK-CMV plasmid (Amersham) containing the DNA sequence coding for protein 20.3 of *Leishmania major.* Gene 20.3 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-22.1: *E. coli XL1-Blue* bacteria are transformed by the pBK-CMV plasmid (Amersham) containing the DNA sequence coding for protein 22.1 of *Leishmania major.* Gene 22.1 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

Bp K-26: *E. coli XL1-Blue* bacteria are transformed by the pBK-CMV plasmid (Amersham) containing the DNA sequence coding for protein 26 of *Leishmania major.* Gene 26 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-59: *E. coli XL1-Blue* bacteria are transformed by the pBK-CMV plasmid (Amersham) containing the DNA sequence coding for protein 59 of *Leishmania major.* Gene 59 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-65: *E. coli XL1-Blue* bacteria are transformed by the pBK-CMV plasmid (Amersham) containing the DNA sequence coding for protein 65 of *Leishmania major.* Gene 65 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-77: *E. coli XL1-Blue* bacteria are transformed by the pBK-CMV plasmid (Amersham) containing the DNA sequence coding for protein 77 of *Leishmania major.* Gene 77 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

Bp K-39: *E. coli XL1-Blue* bacteria are transformed by the pBK-CMV plasmid (Amersham) containing the DNA sequence coding for protein 39 of *Leishmania major.* Gene 39 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-68: *E. coli XL1-Blue* bacteria are transformed by the pBK-CMV plasmid (Amersham) containing the DNA sequence coding for protein 68 of *Leishmania major.* Gene 68 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-90: *E. coli XL1-Blue* bacteria are transformed by the pBK-CMV plasmid (Amersham) containing the DNA sequence coding for protein 90 of *Leishmania major.* Gene 90 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-71: *E. coli XL1-Blue* bacteria are transformed by the pBK-CMV plasmid (Amersham) containing the DNA sequence coding for protein 71 of *Leishmania major.* Gene 71 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

Bp K-42: *E. coli XL1-Blue* bacteria are transformed by the pBK-CMV plasmid (Amersham) containing the DNA sequence coding for protein 42 of *Leishmania major.* Gene 42 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-32: *E. coli XL1-Blue* bacteria are transformed by the pBK-CMV plasmid (Amersham) containing the DNA sequence coding for protein 32 of *Leishmania major.* Gene 32 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pMOS-8.2: *E. coli XL1-Blue* bacteria are transformed by the pMOS-Blue plasmid (Amersham) containing the DNA sequence coding for protein 8.2 of *Leishmania major.* Gene 8.2 has been cloned at the *EcoR* V restriction site. The recombinant (transformed) bacteria are resistant to tetracycline and ampicillin. The genes which give resistance to ampicillin and to tetracycline are carried by the recombinant pMOS-Blue plasmid and XL1-Blue bacteria, respectively.

pBK-21: E. *coli XL1-Blue* bacteria are transformed by the pBK-CMV plasmid (Amersham) containing the DNA sequence coding for protein 21 of *Leishmania major.* Gene 21 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-57: *E. coli XL1-Blue* bacteria are transformed by the pBK-CMV plasmid (Amersham) containing the DNA sequence coding for protein 57 of *Leishmania major.* Gene 57 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-74: *E. coli XL1-Blue* bacteria are transformed by the pBK-CMV plasmid (Amersham) containing the DNA sequence coding for protein 74 of *Leishmania major.* Gene 74 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

pBK-13: *E. coli XL1-Blue* bacteria are transformed by the pBK-CMV plasmid (Amersham) containing the DNA sequence coding for protein 13 of *Leishmania major.* Gene 13 has been cloned at the *Xhol* and *EcoR* I restriction sites. The recombinant (transformed) bacteria are resistant to tetracycline and kanamycin. The genes which give resistance to kanamycin and to tetracycline are carried by the recombinant pBK-CMV plasmid and XL1-Blue bacteria, respectively.

The above biological material is submitted to the following conditions.

### 1- Culture conditions

### Culture medium

The recombinant bacteria are grown either on a liquid culture medium, or on a solid culture medium.
The liquid culture medium is Luria-Broth (LB), and comprises: 1% (w/v) Bactotryptone; 0.5% (w/v) yeast extract and 1% (w/v) NaCl. The pH of medium is set to 7. This medium is appropriate for the multiplication of the pMOS-Blue plasmids or the pBK-CMV plasmids containing the genes repectively described above.
The solid culture medium is Luria-Broth-Agar (LB-Agar), and comprises 1% (w/v) bactotryptone; 0.5% (w/v) yeast extract; 1 % (w/v) NaCl and 1.5% (w/v) agar. This medium is essentially appropriate for growing recombinant bacteria, which have been frozen at -80°C. These two media are sterilized at 120°C for 20 minutes, immediately after having being prepared.

### Inoculation conditions

The recombinant bacteria, which have been frozen at -80°C, are placed on a dish containing LB-Agar solid culture medium, supplemented with 15 microgrammes/mL of tetracycline, and 50 microgrammes/mL of ampicillin (for the cells containing recombinant pMOS-Blue plasmids or 15 microgrammes/mL of tetracycline, and 50 microgrammes/mL of kanamycin) (for the cells containing recombinant pBK-CMV plasmids) for 18 hours at 37°C. A colony is then isolated, and placed for incubation in 5mL of LB, supplemented with the same antibiotics. The culture is conducted for 18 hours at 37°C.

### Incubation (temperature, atmosphere, stirring, illumination)

The culture is made at a temperature 37°C under stirring at 200 revolutions/min (SANYO Orbital incubator). The culture is made at ambient temperature, and under natural light.

### Conditions for storage

The recombinant bacteria can be stored by freezing at -80°C in a LB medium, supplemented with 10% glycerol. The cell concentration can be of about 10⁹ cfu/mL.

### 2- Activities to be checked to confirm the viability of the microorganism

The XL1-Blue bacteria, which have been frozen at -80°C and which contain the recombinant pMOS-Blue plasmid or the recombinant pBK-CMV plasmid, are placed on a LB-Agar medium, supplemented with 15 microgrammes/mL of tetracycline, and of 50 microgrammes/mL of ampicillin, or 15 microgrammes/mL of tetracycline (for the cells containing the pMOS-Blue plasmids), and of 50 microgrammes/mL of kanamycin (for the cells containing the pBK-CMV plasmids), for 18h at 37°C. The viability of the frozen bacteria is checked by the presence or absence of bacterial colonies.

### 3- Additional information

### Origin of the microorganism

The non-recombinant E. coli XL1-Blue bacteria have been bought from Stratagen (La Jolla, California, USA). The recombinant bacteria containing the plasmid of interest have been made in our laboratory, i.e., the *Laboratoire d'Immunologie et de Vaccinologie Mo*/*éculaire* (LIVGM) of the Pasteur Institute of Tunis (Tunisia).

### EXAMPLE

### Identification of excreted/secreted proteins by Leishmania major parasite

### Materials and methods

### Parasites culture.

A High virulent isolate of *L. major* (zymodeme MON25; MHOM/TN/94/GLC94), obtained from human ZCL lesion was used in this study [Kébaier, 2001]. Parasites were cultivated on NNN medium at 26°C and were then progressively adapted to RPMI 1640 medium (Sigma, St. Louis, Mo.) containing 2 mmol/ml L-glutamine, 100 U/ml penicillin, 100 µg/ml streptomycin, and 10% heat-inactivated foetal calf serum (complete medium). Promastigotes collected at the logarithmic-growth phase culture were adjusted to 10⁶ parasites/ml in a constant volume and further incubated at 26°C. The stationary phase was reached after 6 days with parasite concentration of 8 x 10⁷ parasites/ml. Stationary phase promastigotes were used for proteins labeling and preparation of excreted/secreted proteins of *L. major.*

### Preparation of L. major excreted-secreted protein (LMES).

Confluent parasites from six culture flasks of *L*. *major* stationary phase promastigotes were incubated overnight in RPMI 1640 complete medium pH 7.6 at 35°C under 5% CO₂ atmosphere. To eliminate any contaminant protein of foetal calf serum, parasites were washed six times with RPMI 1640 media. Parasites were then resuspended at 2 x 10⁷ parasites/ml in RPMI minimum media pH 5.5 and incubated for 6 hours at 35°C under 5% CO₂ atmosphere. The viability of the parasites after 6 hours of incubation was assessed by the Trypan blue exclusion test of cell viability [Berredo-Pinho, 2001] and found to be over 97%. Following this incubation, the supernatant containing secreted/excreted proteins (LMES) was collected by centrifugation at 4000 x g for 20 min at 4°C then lyophilized using a speed-vaccum concentrator (Savant, Holbrook, NY). Before use, proteins were reconstituted with distilled water. The amounts of proteins in LMES were determined by the Lowry assay.

### Generation of rabbit anti-LMES sera.

One rabbit was immunized by intramuscular (IM) route with 250 µg of the LMES emulsified in incomplete Freund's adjuvant (Sigma, Steinheim, Germany). The rabbit received one additional IM injection with the same amount of protein emulsified in incomplete Freund's adjuvant by the intramuscular route 15 days after the first injection. One month later, a final injection with 250 µg of the LMES without adjuvant was administered by intradermal injections in eight different sites. The rabbit was bled starting 10 days after the final injection. The rabbit immune sera raised against excreted-secreted proteins were tested then used for the immunoscreening of *L. major* cDNA library and immunoprecipitation experiments.

### Proteins labeling and separation.

Labeling experiments were performed in MEM-based methionine free media (Gibco BRL, Paisley, Scotland) titrated to pH 5.5 with 20 mM succinic acid [2]. Promatigotes (1 x 10⁸ cells) from *L. major* stationary phase were preincubated for one hour at different temperature and pH conditions (35°C, pH 5.5 and 26°C, pH 7.6) in complete medium. Parasites were then labeled by further incubation for another 6 hours in the same medium containing 20 µCi/ml of [³⁵S] methionine (specific activity,>1,000 Ci/mmol; Amersham, UK). Following labeling, the supernatant containing excreted/secreted proteins was collected by centrifugation at 4,000 x *g* for 20 min at 4°C and treated with a mixture of protease inhibitors (containing pepstatin, leupeptin and PMSF, Boehringer, Mannheim, Germany). The radiolabled proteins released in the supernatants were concentrated to 1/10 of the initial volume by centrifugation with nominal 10.000-molecular-weight-cutoff Centricon YM-10 tubes (Millipore, Bedford, Mass.) as described by the manufacturer. Ten µl of radiolabled concentrated supernatants were resuspended in 1x SDS sample buffer, heated at 95°C for 10 min and analyzed by SDS-PAGE. The gel was dried, exposed to X-OMAT^{™} films (Eastman Kodak Co, Rochester, NY.) and developed by immersion in X-ray film processing (AGFA-Gevaert, Mortsel, Belgium).

### Immunoprecipitation of labeled proteins.

The [³⁵S] methionine radiolabeled excreted-secreted proteins were immunoprecipitated by rabbit antiserum raised against LMES. Prior to immunoprecipitation, concentrated *L. major* supernatants were incubated in NP-40 buffer (50 mM Tris-Hcl [pH 7.5], 150 mM NaCl, 0.5% [v/v] Nonidet P-40) in the presence of a mixture of protease inhibitors (Boehringer, Mannheim, Germany). Insoluble fraction was removed from the supernatant by centrifugation at 12.000 x *g* for 20 min at 4°C. The supernatant fraction was incubated overnight at 4°C with 20µl of antiserum to LMES. Immune complexes were adsorbed on protein A-Sepharose CL4B beads (Pharmacia, Uppsala, Sweden) by incubation at 4°C with constant rocking for two hours. Sepharose CL4B beads were recovered by centrifugation, washed three times in NP-40 buffer, and separated by SDS-PAGE followed by autoradiography.

### Immunoscreening of cDNA library of L. major promastigote.

An oligo (dT)-primed cDNA library from *L. major* promastigote poly(A)+RNA was constructed in ZAP II Phage expression vector according to the instructions of the manufacturer (Stratagene, La Jolla, Calif.). The resultant library was estimated to contain 1.48 10⁸ plaque forming units per ml [4]. A lawn of XL1-MRF' host cells infected with about 1× 10⁴ PFU of the phage stock was prepared on a 82-mm plates and incubated for 8 h at 37°C. The lawn was then overlaid with a Hybond^{™}-C nitrocellulose membrane disc (Amersham-Life science, UK) presoaked in 10 mM isopropyl-^{β}-D-thiogalactopyranoside (IPTG) for induction of protein expression by further incubation at 37°C for overnight. The plate and membrane were indexed and oriented for matching corresponding plate and membrane position. Approximately 5 × 10⁵ plaques were screened. After transfer, membranes were then washed five times in TBS-T (20 mM Tris-Hcl [pH 7.5], 150 mM NaCl, 0.05% [v/v] Tween 20) and blocked in 5% (w/v) nonfat dried milk-TBS-T at room temperature for 1 hour. Membranes from the expression library were incubated with antiserum to LMES diluted to 1:500 in blocking solution for 2 h with rocking at room temperature and with pre-immune serum to 1:500 as control followed by three washes in TBS-T. A secondary antibody of peroxidase-conjugated goat anti-rabbit IgG (Amersham-Pharmacia, UK) diluted to 1:2,000 in TBS-T was added to the membranes and allowed to incubate for 1 h at room temperature. After a final wash, colorimetric detection was performed using diaminobenzidine tetrahydrochloride (Sigma, St. Louis, MO) in 50 mM Tris-Hcl [pH 7.6] containing 0.03 % hydrogen peroxide (Sigma, St. Louis, MO). The reaction was stopped by washing two times in distilled H₂O. Positive plaques were cored out, and recombinant phage was eluted in 500 µl of SM buffer (50 mM Tris-HCl [pH 7.5] 100 mM NaCl, 10 mM MgSO₄) containing 2% chloroform (Stratagene manual). These were replated at about 50 to 200 PFU on 82-mm plates for secondary and tertiary screenings using the same anti-LMES sera. Positive recombinant phage clones from tertiary screenings were subjected to pBK-CMV phagemid vector excision from the ZAP Express vector using the ExAssist helper phage according to the manufacturer protocol. The recombinant plasmids DNA were purified with an anion-exchange silica-gel membrane (Qiagen GmbH, Germany) as recommended by the manufacturer.

### Sequence analysis of cDNA inserts, databases and software.

The recombinant plasmids were DNA sequenced using the forward T3 (5'-aattaaccctcactaaaggg-3') and the backward T7 ( 5-'gtaatacgactcactatagggc-3') vectors primers (Stratagene manual) by the dideoxy chain terminator method using fluorescent BigDye^{™} terminators in ABI PRISM 377-A Stretch DNA sequencer (Perkin-Elmer). The nucleotide sequence of the isolated cDNA clones were compared with known nucleic acid sequences (Blast and *L*. *major* OmniBlast ) and amino acid sequences were deduced (Blast, Scanprosite and PSORT II) in various databases (NCBI, EBI, Sanger Institute and SMART). The presence and location of signal peptide cleavage sites in the amino acid sequences of the translated cDNAs were predicted using SignalP server (http://www.cbs.dtu.dk/services/SignalP/).

### Preparation of E. coli crude extracts and Western blot analysis.

Overnight cultures of XL1-Blue MRF' harbouring the recombinant plasmide pBK-CMV were diluted to 1:100 in fresh Lauria Broth (Amersham-Pharmacia, UK) containing 50 µg of Kanamycin per ml and grown with vigorous shaking to an optical density at 600 nm (OD₆₀₀) of 0.6. Isopropyl-^{β}-D-thiogalactopyranoside (IPTG) was added to the culture to a final concentration of 1 mM, and the induced culture was grown for an additional 4 hours. Crude cell extracts were prepared by washing cells with TE (10 mM Tris-Hcl [pH 7.5], 1 mM EDTA), resuspending them in 1x SDS sample buffer, and heating them at 95°C for 10 min. Protein was separated by sodium dodecyl sulfate-18% polyacrylamide gel electrophoresis (SDS-PAGE) and transferred onto nitrocellulose membranes (Amersham-Pharmacia, UK) by Western blotting using the Bio-Rad TransBlotter (according to the manufacturer's protocol). After transfer, membranes were blocked for 1 hour in TBS-T buffer containing 3% (w/v) nonfat dried milk (blocking solution) at room temperature for 1 hour. Incubation with antiserum to LMES diluted to 1:500 in blocking solution and with pre-immune serum diluted to 1:500 as control was carried out with rocking for1h at room temperature. The nitrocellulose membranes were then washed three times with TBS-T before incubation with goat anti-rabbit IgG secondary antibody conjugated to peroxidase (1:1000 in 3% nonfat dried milk-TBS-T) for another 1 h at room temperature. The nitrocellulose membranes were again washed three times in TBS-T, and revealed using DAB-H₂O₂ substrate as described previously.

### Results and Discussion

### Characterization of leishmania major excreted-secreted antigens.

In order to identify proteins that *Leishmania* parasites possibly release into the phagolysosomal vacuole of host macrophages, stationary phase promastigotes were exposed to conditions that partially mimic the macrophages vacuole environment. Therefore, promastigotes from *L. major* isolates GLC94 were first cultured in complete medium at pH7.5 and at 26°C until stationary phase was reached. Parasites were in vivo labeled by ³⁵S methionine incubation at pH5.5 and at 35°C. Control cultures were maintained at pH 7.5 and at 26°C. A short incubation period of only 6 hours was used to avoid excessive cell death and proteins release from dead parasites. Radiolabeled proteins released in the culture media were concentrated using centricon YM-10 Centrifugal Filter and analyzed by SDS-PAGE (figure 1 lanes 1 and 3). As expected, several proteins were detected in both culture conditions. Interestingly, the pattern of these proteins were different. At pH 7.5 and at 26°C, few proteins were observed with 3 major proteins migrating at a molecular weight of 70kDa, 66kDa and 50kDa (figure 1, lane 1). In contrast, at pH5,5 and 35°C, several proteins were detected ranging from 15kDa to 70kDa MW (figure 1, lane 2). Interestingly, two proteins with a molecular weight of approximatively 50kDa and 30kDa appear to be highly induced by these culture conditions.

In order to characterize the observed proteins, the rabbit polyclonal antiserum raised against excreted-secreted products of *L. major* parasites (anti-LMES) was used to immunoprecipitate them. As shown in the figure 1, at pH5,5 and 35°C, anti-LMES reacts essentially with a 50kDa protein.

To identify *Leishmania* excreted antigens, anti-LMES was used to isolate clones from a cDNA expression library from *L. major* promastigotes . From a screen of approximately 5 x 10⁵ plaques, 52 immunoreactive clones were isolated and sequenced. The analysis of the isolated sequences reveal that some of them were identical and therefore a total of 34 clones were different. The sequence search for homology of the isolated clones with known sequences carried out using many bioinformatic programs; Blast from NCBI and EBI (hftp://www.ncbi.nlm.nih.gov, http://www.ebi.ac.uk) and *L. major* OmniBlast form the Sanger Institute (http://www.sanger.ac.uk) Server programs for both nucleotide and peptide revealed that 62% of cDNA clones displayed significant homologies with known genes of proteins from *Leishmania* and other species (table 1). Potential open reading frames (ORFs) were identified using traduction multiple (http://www.infobiogen.fr/) and proteins sequence analysis were carried out using Blast from NCBI and EBI, SMART (http://smart.embi-heidelberg.de), Scanprosite (http://au.expasy.org), PSORT II (http://psort.nibb.ac.jp) and SignalP server (http://www.cbs.dtu.dk/services/SignalP).

### LmPDI

PDI is a member of the thioredoxin superfamily which is composed of several redox proteins playing a key role in disulfide bond formation, isomerisation, and reduction within the ER, and it displays chaperone activity [Ferrari, 1999; Wilkinson, 2004]. These molecules are essential for assisting unfolded or incorrectly folded proteins to attain their native state [Ferrari, 1999; Wilkinson, 2004]. Different cellular localizations were attributed to the Protein Disulfide Isomerase (PDI) family. First, in the lumen of the endoplasmic reticulum via its ER retention signal KDEL, second, in the plasma membrane and finally, released in the extracellular space [Turano, 2002; Geldof, 2003]. The *Leishmania major* protein disulfide isomerase (LmPDl) has been recently described as a putative virulence protein of the parasite [Ben Achour, 2002]. In fact, the LmPDl gene is predominantly expressed, at both mRNA and protein levels, in highly virulent isolates than in lower virulent isolates. In addition, specific PDI inhibitors ablated the enzymatic activity of the recombinant protein LmPDl and profoundly affected parasite growth in vitro and in vivo. However, the mechanism by which excreted/secreted LmPDl may affect parasite virulence is presently unknown.

### PSA-2

The promastigote Surface Antigen-2 (PSA-2) complex proteins are protozoan specific proteins. The exact function of the PSA-2 protein is not known but its localization, expression and immnogenicity were fully characterized in *Leishmania. Leishmania* PSA-2 is a family of glycosylinositol phospholipid-anchored polypeptides. Interestingly, several studies have described PSA-2 proteins as excreted/secreted proteins [Symons, 1994; Webb, 1998]. In addition, the genes of PSA-2 family are differentially expressed during the parasite life cycle [Handman, 1995; Jimenez-Ruiz, 1998]. Some of them are more expressed in the promastigotes stationary phase and may be involved in the metacyclogenesis. Other members of this family are essentially expressed by *Leishmania* amastigotes suggesting that they may exert their function during the intracellular stage of the parasite. The immunogenicity of the PSA-2 complex proteins was well studied in human and in the mouse model of experimental leishmaniasis, it was demonstrated that the PSA-2 protein induces a Th1 type of response in both patients with self-resolved CL and in infected mice [Handman, 1995; Kemp, 1998]. In addition, the PSA-2 protein induces a significant protection of mice against a parasite challenge using virulent *Leishmania* [Handman, 1995].

### HSP-70

The heat shock proteins 70 are highly conserved among different species (Archaea, eubacteria and eukaryotes) and are highly represented under conditions of cellular stress. The HSP-70 display chaperone activity and are therefore involved in protein folding and transport [Bassan, 1998]. Interestingly, recent studies showed that these proteins specifically inhibit the cellular apoptosis [Garrido, 2003]. Interestingly, the HSP-70 was described as an excreted/secreted protein [Pockley, 1998; 1999; Rea, 2001].

In *Leishmania,* the hsp 70 gene was well characterized and as reported for hsp70 genes from different species, its expression increased, in vitro and in vivo, in response to a heat and/or oxidant stress [Garlpati, 1999]. This response may be involved in parasite survival and proliferation into mammalian host cells. It has also been described that the *trypanosmatidae* Hsp70 proteins displayed high immunostimulatory properties. Recently, Planelles et al, (2001) showed that the DNA immunization of mice with *Trypanosoma cruzi* KMP11-HSP70 fused genes elicited both an immunoglobulin G2a long-lasting humoral immune response against KMP11 protein and activation of CD8+ cytotoxic T lymphocytes specific to KMP-11. Moreover, protection against the parasite challenge was observed in mice immunized with the chimeric gene [Planelles, 2001]. In *Leishmania,* the nuclease P4 fused with the Hsp70 (P4/Hsp70) was proposed as a vaccine candidate [Campbell, 2003]. It was demonstrated that the P4/Hsp70 induced a Th1 cytokine profile in BALB/c mice immunized by a DNA vaccine containing P4/Hsp70 fused genes. In addition, the DNA vaccine encoding P4/HSP70 induced significant protection against L. *major* challenge. It was reported by Rico et al (2002) that *Leishmania* heat shock proteins Hsp70 and Hsp83, are potent mitogens for murine splenocytes. In vitro incubation of spleen cells with the *Leishmania* Hsps leads to the expansion of B220-bearing populations, suggesting a direct effect of these proteins on B lymphocytes an indication that the MBP-Hsp70 and MBP-Hsp83 recombinant proteins behave as T cell-independent mitogens of B cells. Furthermore, both proteins were able to induce proliferation on B cell populations purified from BALB/c spleen [Rico, 2002].

### Cathepsin L-like protease

The cathespin L proteins are members of the papaïn superfamily and are expressed by several species. In *Faciola Hepatica* parasite the cathepsin L protease was well studied and it was demonstrated that this protein is excreted/secreted and involved in the virulence of the parasite [Collins, 2004]. Recently, it was shown that it may constitute a good vaccine candidate [Dalton, 2003; Harmsen,2004]. In *Leishmania,* the cysteine proteinases have been also described as virulence factors [Motram, 1996; Matlashewki, 2001]. The gene of the cathepsin L-like proteinase is stage regulated with high expression in amastigotes, lower expression in metacyclics and very low in procyclics [Souza, 1994]. These results suggest that this enzyme may play an important role in intracellular survival of the parasite.

### KMP-11

The Kinetoplast Membrane Protein-11 (KMP-11) is a surface glycoprotein of Kinetoplastidae parasites. In *Leishmania,* KMP-11 is tightly associated with lipophosphoglycan (LPG) and contributes to its stability. KMP-11 is expressed in both promastigotes and amastigotes stages at the surface of the parasite [Tolson, 1994; Jardim, 1995]. Mukhopadhyay *et al.* (1998), have been shown that the KMP-11 protein may be involved in *Leishmania* virulence [Mukhopadhyay, 1998]. In addition to its role in the pathogenicity of the parasite, KMP-11 was proposed by different authors as a good vaccine candidate. In fact, it was described to elicit potent lymphoproliferative and antibody responses in leishmaniasis patients or experimentally infected mice [Jensen, 1998; Requena, 2000; Delgado, 2004]. Interestingly, a strong protective effect was observed in mice vaccinated with Langerhans cells pulsed with different *Leishmania* antigens, KMP-11, LACK, PSA-2 and gp63 after a virulent challenge with L. *major* [Berberich, 2003].

### Spermidine synthase

The spermidine synthase protein is involved in the polyamine biosynthetic pathway [Kaiser, 2003]. The spermidine synthase catalyzes the synthesis of spermidine by transfering a propylamine group from decarboxylated S-adenosylmethionine to putrescine. The spermidine synthase is well conserved among several species [Kaiser, 2003]. In protozoa including *Leishmania,* spermidine may play a crucial role in cell proliferation, cell differentiation, and biosynthesis of macromolecules [Kaiser, 2003]. Targeting polyamines of protozoa by chemotherapy may constitute a new way for the identification of new anti-leishmanial drugs [Kaiser, 2003]. In fact recent studies have shown that specific inhibitors of spermidine synthase decrease parasite proliferation [Kaiser, 2003].

### Cytochrome C

Cytochromes *c* can be defined as electron-transfer proteins having one or several haem *c* groups, bound to the protein by one or, more commonly two, thioesther bonds involving sulphydryl groups of cysteine residues. Cyt *c* possesses a wide range of properties and function in a large number of different redox processes [Namslauer, 2004]. This protein is released in the extracellular culture medium in the early steps of cell apoptotisis [Saelens, 2004]. A recent study showed that the induced *Leishmania* apoptosis is accompanied with cytochrome c release from the mitochondria [Akarid, 2004]. Interestingly, cytochrome c of *Mycobacterium tuberculosis* induces IFN-gamma secretion and proliferation of human PBMC from purified protein derivative- (PPD)-positive individuals [Moran, 1999]. Thus it was proposed as a good vaccine candidate.

### Ribosomal proteins and proteins associated with the proteasome

Two kinds of ribosomal proteins family have been detected in the culture medium: those associated with the large subunit of the ribosome (L) and those associated with the small subunit (S). All these proteins are well conserved among eukaryotic and prokaryotic species. It was reported that different ribosomal proteins are released in the culture medium of different pathogens including *Leishmania* [Ouaissi, 2004]. Moreover, the ribosomal protein L7/L12 of *Brucella abortus* was proposed as a good vaccine candidate. In fact, it confers a protection in the mouse model after a virulent challenge [Kurar, 1997; Pontes, 2003]. In *Leishmania,* Probst et al, (2001) using parasite-specific T cell lines derived from an immune donor showed that the ribosomal protein S4 induces high lymphoproliferative responses associated with a secretion of significant amounts of IFN-g [Probst, 2001]. Sequence analysis the *Leishmania* ribosomal proteins did not reveal any signal peptide and thus it is not clear by which mechanism they might be secreted. Two proteins with significant homologies with proteins associated with the proteasome were also released in the culture medium. These proteins may be involved in intracellular proteolytic processes of the parasite. Like ribosomal proteins, these proteins lack signal peptide and therefore mechanisms by which these proteins are exported outside the parasite remain to be determined.

### Leishmania proteins that did not display any homologies with known proteins

Thirteen proteins detected in the culture medium did not correspond to proteins described in sequences libraries. However a majority of these proteins displayed very specific conserved functional domains and almost all contain a signal peptide. Additional studies are in progress to characterize these proteins.

### REFERENCES:

Akarid K, Arnoult D, Micic-Polianski J, Sif J, Estaguier J, Ameisen JC. Leishmania major-mediated prevention of programmed cell death induction in infected macrophages is associated with the repression of mitochondrial release of cytochrome c. J Leukoc Biol. 2004 Jul;76(1):95-103.
Bassan M, Zamostiano R, Giladi E et al. The identification of secreted heat shock 60- like protein from rat glial cells and a human neuroblastoma cell line. Neurosci Lett 1998;250: 37-40.
Berberich C, Ramirez-Pineda JR, Hambrecht C, Alber G, Skeiky YA, Moll H. Dendritic cell (DC)-based protection against an intracellular pathogen is dependent upon DC- derived IL-12 and can be induced by molecularly defined antigens. J Immunol. 2003 Mar 15;170(6):3171-9.
Berredo-Pinho M, Peres-Sampaio CE, Chrispim PP, Belmont-Firpo R, Lemos AP, Martiny A, Vannier-Santos MA, Meyer-Fernandes JR. A Mg-dependent ecto-ATPase in Leishmania amazonensis and its possible role in adenosine acquisition and virulence. Arch Biochem Biophys. 2001 Jul 1;391(1):16-24.
Campbell K, Diao H, Ji J, Soong L. DNA immunization with the gene encoding P4 nuclease of Leishmania amazonensis protects mice against cutaneous Leishmaniasis. Infect Immun. 2003 Nov;71(11):6270-8.
Collins PR, Stack CM, O'Neill SM, Doyle S, Ryan T, Brennan GP, Mousley A, Stewart M, Maule AG, Dalton JP, Donnelly S. Cathepsin L1, the major protease involved in liver fluke (Fasciola hepatica) virulence: propetide cleavage sites and autoactivation of the zymogen secreted from gastrodermal cells. J Biol Chem. 2004 ;279(17):17038-46.
Dalton JP, Neill SO, Stack C, Collins P, Walshe A, Sekiya M, Doyle S, Mulcahy G, Hoyle D, Khaznadji E, Moire N, Brennan G. Mousley A, Kreshchenko N, Maule AG, Donnelly SM. Fasciola hepatica cathepsin L-like proteases: biology, function, and potential in the development of first generation liver fluke vaccines. Int J Parasitol. 2003 Sep 30;33(11):1173-81. Review.
Daryani A, Hosseini AZ, Dalimi A. Immune responses against excreted/secreted antigens of Toxoplasma gondii tachyzoites in the murine model. Vet Parasitol. 2003 Apr 18;113(2):123-34.
Delgado G, Parra-Lopez CA, Vargas LE, Hoya R, Estupinan M, Guzman F, Torres A, Alonso C, Velez ID, Spinel C, Patarroyo ME. Characterizing cellular immune response to kinetoplastid membrane protein-11 (KMP-11) during Leishmania (Viannia) panamensis infection using dendritic cells (DCs) as antigen presenting cells (APCs). Parasite Immunol. 2003;25(4):199-209.
Garlapati S, Dahan E, Shapira M. Effect of acidic pH on heat shock gene expression in Leishmania. Mol Biochem Parasitol. 1999 May 15;100(1):95-101.
Garrido C, Schmitt E, Cande C, Vahsen N, Parcellier A, Kroemer G. HSP27 and HSP70: potentially oncogenic apoptosis inhibitors. Cell Cycle. 2003 Nov-Dec;2(6):579-84.
Geldhof P, Vercauteren I, Knox D, Demaere V, Van Zeveren A, Berx G, Vercruysse J, Claerebout E. Protein disulphide isomerase of Ostertagia ostertagi: an excretory-secretory product of L4 and adult worms? Int J Parasitol. 2003 Feb;33(2):129-36.
Handman E, Osborn AH, Symons F, van Driel R, Cappai R. The Leishmania promastigote surface antigen 2 complex is differentially expressed during the parasite life cycle. Mol Biochem Parasitol. 1995 Nov;74(2):189-200.
Handman E, Symons FM, Baldwin TM, Curtis JM, Scheerlinck JP. Protective vaccination with promastigote surface antigen 2 from Leishmania major is mediated by a TH1 type of immune response. Infect Immun. 1995 Nov;63(11):4261-7.
Harmsen MM, Cornelissen JB, Buijs HE, Boersma WJ, Jeurissen SH, van Milligen FJ. Identification of a novel Fasciola hepatica cathepsin L protease containing protective epitopes within the propeptide. Int J Parasitol. 2004 May;34(6):675-82.
Jensen AT, Gasim S, Ismail A, Gaafar A, Kurtzhals JA, Kemp M, EI Hassan AM, Kharazmi A, Theander TG. Humoral and cellular immune responses to synthetic peptides of the Leishmania donovani kinetoplastid membrane protein-11. Scand J Immunol. 1998 Jul; 48(1):103-9.
Jimenez-Ruiz A, Boceta C, Bonay P, Requena JM, Alonso C. Cloning, sequencing, and expression of the PSA genes from Leishmania infantum. Eur J Biochem. 1998 Jan 15;251(1-2):389-97.
Kaiser AE, Gottwald AM, Wiersch CS, Maier WA, Seitz HM. Spermidine metabolism in parasitic protozoa--a comparison to the situation in prokaryotes, viruses, plants and fungi. Folia Parasitol (Praha). 2003 Mar;50(1):3-18.
Kemp M, Handman E, Kemp K, Ismail A, Mustafa MD, Kordofani AY, Bendtzen K, Kharazmi A, Theander TG. The Leishmania promastigote surface antigen-2 (PSA-2) is specifically recognised by Th1 cells in humans with naturally acquired immunity to L. major.
Kurar E, Splitter GA. Nucleic acid vaccination of Brucella abortus ribosomal L7/L12 gene elicits immune response. Vaccine. 1997 Dec;15(17-18):1851-7.
Matlashewski G. Leishmania infection and virulence. Med Microbiol Immunol (Berl). 2001 Nov;190(1-2):37-42..
Mendez S, Belkaid Y, Seder RA, Sacks D. Optimization of DNA vaccination against cutaneous leishmaniasis. Vaccine. 2002 Nov 1;20(31-32):3702-8.
Moran AJ, Doran JL, Wu J, Treit JD, Ekpo P, Kerr VJ, Roberts AD, Orme IM, Galant S, Ress SR, Nano FE. Identification of novel immunogenic Mycobacterium tuberculosis peptides that stimulate mononuclear cells from immune donors.
Mottram JC, Souza AE, Hutchison JE, Carter R, Frame MJ, Coombs GH. Evidence from disruption of the lmcpb gene array of Leishmania mexicana that cysteine proteinases are virulence factors.
Mukhopadhyay S, Sen P, Maiumder HK, Roy S. Reduced expression of lipophosphoglycan (LPG) and kinetoplastid membrane protein (KMP)-11 in Leishmania donovani promastigotes in axenic culture. J Parasitol. 1998 Jun;84(3):644-7.
Mustafa AS. Development of new vaccines and diagnostic reagents against tuberculosis. Mol Immunol. 2002 Sep;39(1-2):113-9.
Namslauer A, Brzezinski P. Structural elements involved in electron-coupled proton transfer in cytochrome c oxidase. FEBS Lett. 2004 Jun 1;567(1):103-10.
Saelens X, Festiens N, Vande Walle L, van Gurp M, van Loo G, Vandenabeele P. Toxic proteins released from mitochondria in cell death. Oncogene. 2004 Apr 12;23(16):2861-74.
Ouaissi A, Ouaissi M, Tavares J, Cordeiro-Da-Silva A. Host Cell Phenotypic Variability Induced by Trypanosomatid-Parasite-Released Immunomodulatory Factors: Physiopathological Implications. J Biomed Biotechnol. 2004;2004(3):167-174.
Pockley AG, Shepherd J, Corton JM. Detection of heat shock protein 70 (Hsp70) and anti-Hsp70 antibodies in the serum of normal individuals. Immunol Invest 1998;27: 367-77.
Pockley AG, Bulmer J, Hanks BM, Wright BH. Identification of human heat shock protein 60 (Hsp60) and anti-Hsp60 antibodies in the peripheral circulation of normal individuals. Cell Stress Chaperones 1999;4: 29-35
Pontes DS, Dorella FA, Ribeiro LA, Miyoshi A, Le Loir Y, Gruss A, Oliveira SC, Langella P, Azevedo V. Induction of partial protection in mice after oral administration of Lactococcus lactis producing Brucella abortus L7/L12 antigen. J Drug Target. 2003;11 (8-10):489-93.
Planelles L, Thomas MC, Alonso C, Lopez MC. DNA immunization with Trypanosoma cruzi HSP70 fused to the KMP11 protein elicits a cytotoxic and humoral immune response against the antigen and leads to protection. Infect Immun. 2001 Oct;69(10):6558-63.
Prigione I, Facchetti P, Lecordier L, Deslee D, Chiesa S, Cesbron-Delauw MF, Pistoia V. Tcell clones raised from chronically infected healthy humans by stimulation with Toxoplasma gondii excretory-secretory antigens cross-react with live tachyzoites: characterization of the fine antigenic specificity of the clones and implications for vaccine development. J Immunol. 2000 Apr 1;164(7):3741-8.
Probst P, Stromberg E, Ghalib HW, Mozel M, Badaro R, Reed SG, Webb JR. Identification and characterization of T cell-stimulating antigens from Leishmania by CD4 T cell expression cloning. J Immunol. 2001 Jan 1;166(1):498-505.
Pym AS, Brodin P, Mailessi L, Brosch R, Demangel C, Williams A, Griffiths KE, Marchal G, Leclerc C, Cole ST. Recombinant BCG exporting ESAT-6 confers enhanced protection against tuberculosis. Nat Med. 2003 May;9(5):533-9.
Ramirez JR, Gilchrist K, Robledo S, Sepulveda JC, Moll H, Soldati D, Berberich C. Attenuated Toxoplasma gondii ts-4 mutants engineered to express the Leishmania antigen KMP-11 elicit a specific immune response in BALB/c mice.Vaccine. 2001 Nov 12;20(3-4):455-61.
Rea IM, McNerlan S, Pockley AG. Serum heat shock protein and anti-heat shock protein antibody levels in aging. Exp Gerontol 2001;36: 341-52
Requena JM, Soto M, Doria MD, Alonso C. Immune and clinical parameters associated with Leishmania infantum infection in the golden hamster model. Vet Immunol Immunopathol. 2000 Oct 31;76(3-4):269-81.
Rico AI, Girones N, Fresno M, Alonso C, Requena JM. The heat shock proteins, Hsp70 and Hsp83, of Leishmania infantum are mitogens for mouse B cells. Cell Stress Chaperones. 2002 Oct;7(4):339-46.
Shams H, Klucar P, Weis SE, Lalvani A, Moonan PK, Safi H, Wizel B, Ewer K, Nepom GT, Lewinsohn DM, Andersen P, Barnes PF. Characterization of a Mycobacterium tuberculosis peptide that is recognized by human CD4+ and CD8+ T cells in the context of multiple HLA alleles. J Immunol. 2004 Aug 1;173(3):1966-77.
Souza AE, Bates PA, Coombs GH, Mottram JC. Null mutants for the Imcpa cysteine proteinase gene in Leishmania mexicana. Mol Biochem Parasitol. 1994 Feb;63(2):213-20.
Symons FM, Murray PJ, Ji H, Simpson RJ, Osborn AH, Cappai R, Handman E. Characterization of a polymorphic family of integral membrane proteins in promastigotes of different Leishmania species. Mol Biochem Parasitol. 1994 Sep;67(1):103-13.
Turano C, Coppari S, Altieri F, Ferraro A. Proteins of the PDI family: unpredicted non-ER locations and functions. J Cell Physiol. 2002 Nov. 193(2): 154-63.
Webb JR, Campos-Neto A, Ovendale PJ, Martin Ti, Stromberg EJ, Badaro R, Reed SG. Human and murine immune responses to a novel Leishmania major recombinant protein encoded by members of a multicopy gene family. Infect Immun. 1998 Jul;66(7):3279-89.
Wilkinson B, Gilbert HF. Protein disulfide isomerase. Biochim Biophys Acta. 2004 Jun 1;1699(1-2):35-44.

## Claims

1. An isolated polynucleotide comprising a sequence encoding an excreted/secreted polypeptide of *Leishmania major,* said sequence comprising a nucleotide sequence substantially identical to the sequence of SEQ ID NO 1 .

2. An isolated or purified excreted/secreted polypeptide of *Leishmania major,* said polypeptide comprising an amino acid sequence substantially identical to the sequence of SEQ ID NO: 35.

3. An immunogenic composition generating an immune response against a leishmaniasis, comprising a polynucleotide as defined in claim 1 or a polypeptide as defined in claim 2, and an acceptable carrier.

4. The immunogenic composition of claim 3, wherein said immune response generate a cellular and/or humoral response.

5. The immunogenic composition of claim 3, wherein said immune response generate a cellular response.

6. A vaccine composition generating a protecting response against a leishmaniasis, comprising a polynucleotide as defined in claim 1 or a polypeptide as defined in claim 2, and an acceptable carrier.

7. An antibody obtainable by the immunization of an animal with a polypeptide as defined in claim 2.

8. An expression or a cloning vector containing a polynucleotide of claim 1.

9. A composition as defined in any one of claims 3 to 6 or of an antibody as defined in claim 7, for use in the prevention and/or treatment of a patient against an infection with a *Leishmania* major strain.

10. A method for identifying an excreted/secreted polypeptide of a *Leishmania major* strain, comprising *in vitro* cultivating Leishmania promastigotes under pH and temperature conditions naturally found in a host cell infected by a *Leishmania major* strain.

11. The method of claim 10, wherein the pH is about 5.5 and the temperature is about 35°C.

12. Use of a polynucleotide as defined in claim 1 and/or a polypeptide as defined in claim 2, as a target for identifying a molecule capable of preventing a Leishmaniasis.

13. An *in vitro* diagnostic method for the detection of the presence or absence of antibodies indicative of a *Leishmania major* strain, which bind to a polypeptide according to claim 2 to form an immune complex, comprising the steps of
a) contacting said polypeptide with a biological sample for a time and under conditions sufficient to form an immune complex; and
b) detecting the presence or absence of the immune complex formed in a).

14. A diagnostic kit for the detection of the presence or absence of antibodies indicative of a *Leishmania major* strain, comprising:
- a polypeptide according to claim 2;
- a reagent to detect polypeptide-antibody immune complex;
- optionally a biological reference sample lacking antibodies that immunologically bind with said peptide; and
- optionally a comparison sample comprising antibodies which can specifically bind to said peptide;
wherein said polypeptide, reagent, biological reference sample, and comparison sample are present in an amount sufficient to perform said detection .

15. An *in vitro* diagnostic method for the detection of the presence or absence of polypeptides indicative of a *Leishmania major* strain, which bind to an antibody of claim 7 to form an immune complex, comprising the steps of:
a) contacting said antibody with a biological sample for a time and under conditions sufficient to form an immune complex; and
b) detecting the presence or absence of the immune complex formed in a).

16. A diagnostic kit for the detection of the presence or absence of polypeptides indicative of a *Leishmania majorstrain,* comprising:
- an antibody according to claim 7;
- a reagent to detect polypeptide-antibody immune complex;
- optionally a biological reference sample lacking polypeptides that immunologically bind with said antibody; and
- optionally a comparison sample comprising polypeptides which can specifically bind to said antibody;
wherein said antibody, reagent, biological reference sample, and comparison sample are present in an amount sufficient to perform said detection.

17. A transformed or transfected cell that contains a vector as defined in claim 8.

18. A transformed or transfected cell that contains a polynucleotide of claim 1.

19. The cell of claim 18, which is the *Escherichia coli* bacterium filed at the CNCM under accession number I-3394 on February 24 2005.

20. A genetically modified Leishmania strain comprising at least one gene having a sequence comprising a nucleotide sequence substantially identical to the sequence of SEQ ID NO 1, and wherein said at least one gene is inactivated.

21. The genetically modified Leishmania strain of claim 20, wherein the gene is inactivated by knock-out.

22. A genetically modified Leishmania strain comprising at least one gene having a sequence comprising a nucleotide sequence substantially identical to the sequence of SEQ ID NO 1, and wherein said at least one gene is underexpressed compared to a corresponding gene of a wild-type strain of Leishmania.

23. The polypeptide of claim 2, for use in the detection of the presence or absence of lymphatic stimulation in a subject suspected of leishmaniasis, wherein said detection comprises the steps of:
a) obtaining a sample containing T Lymphocytes from said subject;
b) contacting the T lymphocytes with said polypeptide; and
c) detecting the presence or absence of a proliferative response of said T lymphocyte to the polypeptide.

24. The polypeptide of claim 2, for use in the detection of the presence or absence of lymphatic stimulation in a subject suspected of leishmaniasis, wherein said detection comprises the steps of:
a) obtaining a sample containing T Lymphocytes from said subject;
b) contacting the T lymphocytes with said polypeptide; and
c) detecting the presence or absence of cytokines indicative of lymphocytic stimulation.

## Patentansprüche

1. Isoliertes Polynukleotid, umfassend eine Sequenz, die ein exkretiertes /sezerniertes Polypeptid von *Leishmania major* kodiert, wobei die Sequenz eine Nukleotidsequenz umfasst, die im Wesentlichen identisch zu der Sequenz von SEQ ID NO: 1 ist.

2. Isoliertes oder gereinigtes exkretiertes /sezerniertes Polypeptid von *Leishmania major,* wobei das Polypeptid eine Aminosäuresequenz umfasst, die im Wesentlichen identisch zu der Sequenz von SEQ ID NO: 35 ist.

3. Immunogene Zusammensetzung, die eine Immunantwort gegen eine Leishmaniase erzeugt, umfassend ein wie in Anspruch 1 definiertes Polynukleotid oder ein wie in Anspruch 2 definiertes Polypeptid, und einen verträglichen Träger.

4. Immunogene Zusammensetzung nach Anspruch 3, wobei die Immunantwort eine zelluläre und/oder humorale Immunantwort erzeugt.

5. Immunogene Zusammensetzung nach Anspruch 3, wobei die Immunantwort eine zelluläre Antwort erzeugt.

6. Vakzinzusammensetzung, die eine schützende Antwort gegen eine Leishmaniase erzeugt, umfassend ein wie in Anspruch 1 definiertes Polynukleotid oder ein wie in Anspruch 2 definiertes Polypeptid, und einen verträglichen Träger.

7. Antikörper, erhältlich durch die Immunisierung eines Tieres mit einem wie in Anspruch 2 definierten Polypeptid.

8. Expressions- oder Klonierungsvektor, enthaltend ein Polynukleotid gemäß Anspruch 1.

9. Zusammensetzung, wie in einem beliebigen der Ansprüche 3 bis 6 definiert, oder von einem Antikörper wie in Anspruch 7 definiert, zur Verwendung in der Prävention und/oder Behandlung eines Patienten gegen eine Infektion mit einem *Leishmania major-Stamm.*

10. Verfahren zum Identifizieren eines exkretierten /sezernierten Polypeptids eines *Leishmania major-Stamms,* umfassend In-vitro-Kultivieren von promastigoten *Leishmania* unter pH- und Temperaturbedingungen, die natürlicherweise in einer durch einen *Leishmania major-Stamm* infizierten Wirtszelle gefunden werden.

11. Verfahren nach Anspruch 10, wobei der pH etwa 5,5 und die Temperatur etwa 35 °C beträgt.

12. Verwendung eines wie in Anspruch 1 definierten Polynukleotids und/oder eines wie in Anspruch 2 definierten Polypeptids als ein Ziel zum Identifizieren eines Moleküls, das zum Vorbeugen einer Leishmaniase fähig ist.

13. In-vitro-diagnostisches Verfahren zur Detektion der Gegenwart oder Abwesenheit von Antikörpern, die für einen *Leishmania major-Stamm* indikativ sind, die unter Bildung eines Immunkomplexes an ein Polypeptid gemäß Anspruch 2 binden, umfassend die Schritte
a) Inkontaktbringen des Polypeptids mit einer biologischen Probe für eine Zeit und unter Bedingungen, die ausreichend sind, um einen Immunkomplex auszubilden; und
b) Detektieren der Gegenwart oder Abwesenheit des in a) gebildeten Immunkomplexes.

14. Diagnostischer Kit zur Detektion der Gegenwart oder Abwesenheit von Antikörpern, die für einen *Leishmania major-Stamm* indikativ sind, umfassend:
- ein Polypeptid gemäß Anspruch 2;
- ein Reagens, um den Polypeptid-Antikörper-Immunkomplex zu detektieren;
- gegebenenfalls eine biologische Referenzprobe, der Antikörper fehlen, die immunologisch mit dem Peptid binden; und
- gegebenenfalls eine Vergleichsprobe, umfassend Antikörper, die spezifisch das Peptid binden können;
wobei das Polypeptid, Reagens, die biologische Referenzprobe und die Vergleichsprobe in einer Menge vorhanden sind, die ausreichend ist, um die Detektion durchzuführen.

15. Diagnostisches In-vitro-Verfahren zur Detektion der Gegenwart oder Abwesenheit von Polypeptiden, die für einen *Leishmania major-Stamm* indikativ sind, die unter Bildung eines Immunkomplexes einen Antikörper gemäß Anspruch 7 binden, umfassend die Schritte:
a) Inkontaktbringen des Antikörpers mit einer biologischen Probe für eine Zeit und unter Bedingungen, die ausreichend sind, um einen Immunkomplex auszubilden; und
b) Detektieren der Gegenwart oder Abwesenheit des in a) gebildeten Immunkomplexes.

16. Diagnostischer Kit zur Detektion der Gegenwart oder Abwesenheit von Polypeptiden, die für einen *Leishmania major-Stamm* indikativ sind, umfassend:
- einen Antikörper gemäß Anspruch 7;
- ein Reagens, um den Polypeptid-Antikörper-Immunkomplex zu detektieren;
- gegebenenfalls eine biologische Referenzprobe, der Polypeptide fehlen, die immunologisch mit dem Antikörper binden; und
- gegebenenfalls eine Vergleichsprobe, umfassend Polypeptide, die spezifisch an den Antikörper binden können;
wobei der Antikörper, das Reagens, die biologische Referenzprobe und die Vergleichsprobe in einer Menge vorhanden sind, die ausreichend ist, um die Detektion durchzuführen.

17. Transformierte oder transfizierte Zelle, die einen wie in Anspruch 8 definierten Vektor enthält.

18. Transformierte oder transfizierte Zelle, die ein Polynukleotid gemäß Anspruch 1 enthält.

19. Zelle nach Anspruch 18, die das *Escherichia coli*-Bakterium ist, hinterlegt bei der CNCM unter Zugangsnummer 1-3394 am 24. Februar 2005.

20. Genetisch modifizierter *Leishmania-Stamm,* umfassend mindestens ein Gen mit einer Sequenz, umfassend eine Nukleotidsequenz, die im Wesentlichen identisch zu der Sequenz von SEQ ID NO: 1 ist, und wobei das mindestens eine Gen inaktiviert ist.

21. Genetisch modifizierter *Leishmania*-Stamm nach Anspruch 20, wobei das Gen durch Knock-out inaktiviert ist.

22. Genetisch modifizierter *Leishmania*-Stamm, umfassend mindestens ein Gen mit einer Sequenz, umfassend eine Nukleotidsequenz, die im Wesentlichen identisch zu der Sequenz von SEQ ID NO: 1 ist, und wobei das mindestens eine Gen verglichen mit einem entsprechenden Gen eines Wildtyp-Stamms von *Leishmania* unterexprimiert ist.

23. Polypeptid gemäß Anspruch 2, zur Verwendung in der Detektion der Gegenwart oder Abwesenheit von lymphatischer Stimulation in einem Subjekt mit Verdacht auf Leishmaniase, wobei die Detektion die Schritte umfasst:
a) Erhalten einer T-Lymphozyten enthaltenden Probe von dem Subjekt;
b) Inkontaktbringen der T-Lymphozyten mit dem Polypeptid; und
c) Detektieren der Gegenwart oder Abwesenheit einer proliferativen Antwort der T-Lymphozyten gegenüber dem Polypeptid.

24. Polypeptid gemäß Anspruch 2, zur Verwendung in der Detektion der Gegenwart oder Abwesenheit von lymphatischer Stimulation in einem Subjekt mit Verdacht auf Leishmaniase, wobei die Detektion die Schritte umfasst:
a) Erhalten einer T-Lymphozyten enthaltenden Probe von dem Subjekt;
b) Inkontaktbringen der T-Lymphozyten mit dem Polypeptid; und
c) Detektieren der Gegenwart oder Abwesenheit von Zytokinen, die für lymphozytische Stimulation indikativ sind.

## Revendications

1. Polynucléotide isolé comprenant une séquence codant pour un polypeptide excrété/sécrété de *Leishmania major,* ladite séquence comprenant une séquence nucléotidique sensiblement identique à la séquence de SEQ ID NO: 1.

2. Polypeptide excrété/sécrété isolé ou purifié de *Leishmania major,* ledit polypeptide comprenant une séquence d'acides aminés sensiblement identique à la séquence de SEQ ID NO: 35.

3. Composition immunogène générant une réponse immunitaire contre une leishmaniose, comprenant un polynucléotide tel que défini selon la revendication 1 ou un polypeptide tel que défini selon la revendication 2, et un support acceptable.

4. Composition immunogène selon la revendication 3, dans laquelle ladite réponse immunitaire génère une réponse cellulaire et/ou humorale.

5. Composition immunogène selon la revendication 3, dans laquelle ladite réponse immunitaire génère une réponse cellulaire.

6. Composition vaccinale générant une réponse protectrice contre une leishmaniose, comprenant un polynucléotide tel que défini selon la revendication 1 ou un polypeptide tel que défini selon la revendication 2, et un support acceptable.

7. Anticorps pouvant être obtenu par l'immunisation d'un animal avec un polypeptide tel que défini selon la revendication 2.

8. Expression ou vecteur de clonage contenant un polynucléotide selon la revendication 1.

9. Composition telle que définie selon l'une quelconque des revendications 3 à 6 ou d'un anticorps tel que défini selon la revendication 7, pour une utilisation dans la prévention et/ou le traitement d'un patient contre une infection par une souche de *Leishmania major.*

10. Procédé d'identification d'un polypeptide excrété/sécrété d'une souche de *Leishmania major,* comprenant la culture *in vitro* de promastigotes de Leishmania dans des conditions de pH et température trouvées naturellement dans une cellule hôte infectée par une souche de *Leishmania major.*

11. Procédé selon la revendication 10, dans lequel le pH est d'environ 5,5 et la température est d'environ 35°C.

12. Utilisation d'un polynucléotide tel que défini selon la revendication 1 et/ou d'un polypeptide tel que défini selon la revendication 2, comme cible pour l'identification d'une molécule capable de prévenir une leishmaniose.

13. Procédé de diagnostic *in vitro* pour la détection de la présence ou l'absence d'anticorps indicateurs d'une souche de *Leishmania major,* qui se lient à un polypeptide selon la revendication 2 pour former un complexe immun, comprenant les étapes de
a) mise en contact dudit polypeptide avec un échantillon biologique pendant une durée et dans des conditions suffisantes pour former un complexe immun ; et
b) détection de la présence ou l'absence du complexe immun formé en a).

14. Trousse de diagnostic pour la détection de la présence ou l'absence d'anticorps indicateurs d'une souche de *Leishmania major,* comprenant :
- un polypeptide selon la revendication 2 ;
- un réactif pour détecter un complexe immun polypeptide-anticorps ;
- éventuellement un échantillon de référence biologique dépourvu d'anticorps qui se lient immunologiquement audit peptide ; et
- éventuellement un échantillon de comparaison comprenant des anticorps qui peuvent se lier spécifiquement audit peptide ;
dans lequel lesdits polypeptide, réactif, échantillon de référence biologique, et échantillon de comparaison sont présents dans une quantité suffisante pour réaliser ladite détection.

15. Procédé de diagnostic *in vitro* pour la détection de la présence ou l'absence de polypeptides indicateurs d'une souche de *Leishmania major,* qui se lient à un anticorps selon la revendication 7 pour former un complexe immun, comprenant les étapes de :
a) mise en contact dudit anticorps avec un échantillon biologique pendant une durée et dans des conditions suffisantes pour former un complexe immun ; et
b) détection de la présence ou l'absence du complexe immun formé en a).

16. Trousse de diagnostic pour la détection de la présence ou l'absence de polypeptides indicateurs d'une souche de *Leishmania major,* comprenant :
- un anticorps selon la revendication 7 ;
- un réactif pour détecter un complexe immun polypeptide-anticorps ;
- éventuellement un échantillon de référence biologique dépourvu des polypeptides qui se lient immunologiquement audit anticorps ; et
- éventuellement un échantillon de comparaison comprenant des polypeptides qui peuvent se lier spécifiquement audit anticorps ;
dans lequel lesdits anticorps, réactif, échantillon de référence biologique, et échantillon de comparaison sont présents dans une quantité suffisante pour réaliser ladite détection.

17. Cellule transformée ou transfectée qui contient un vecteur tel que défini selon la revendication 8.

18. Cellule transformée ou transfectée qui contient un polynucléotide selon la revendication 1.

19. Cellule selon la revendication 18, qui est la bactérie *Escherichia coli* déposée à la CNCM sous le numéro d'ordre I-3394 le 24 février 2005.

20. Souche de Leishmania génétiquement modifiée comprenant au moins un gène ayant une séquence comprenant une séquence nucléotidique sensiblement identique à la séquence de SEQ ID NO 1, et dans laquelle ledit au moins un gène est inactivé.

21. Souche de Leishmania génétiquement modifiée selon la revendication 20, dans laquelle le gène est inactivé par knock-out.

22. Souche de Leishmania génétiquement modifiée comprenant au moins un gène ayant une séquence comprenant une séquence nucléotidique sensiblement identique à la séquence de SEQ ID NO 1, et dans laquelle ledit au moins un gène est sous-exprimé par rapport à un gène correspondant d'une souche de Leishmania de type sauvage.

23. Polypeptide selon la revendication 2, pour une utilisation dans la détection de la présence ou l'absence d'une stimulation lymphatique chez un sujet soupçonné de leishmaniose, dans lequel ladite détection comprend les étapes de :
a) obtention d'un échantillon contenant des lymphocytes T provenant dudit sujet ;
b) mise en contact des lymphocytes T avec ledit polypeptide ; et
c) détection de la présence ou l'absence d'une réponse proliférative dudit lymphocyte T au polypeptide.

24. Polypeptide selon la revendication 2, pour une utilisation dans la détection de la présence ou l'absence d'une stimulation lymphatique chez un sujet soupçonné de leishmaniose, dans lequel ladite détection comprend les étapes de :
a) obtention d'un échantillon contenant des lymphocytes T provenant dudit sujet ;
b) mise en contact des lymphocytes T avec ledit polypeptide ; et
c) détection de la présence ou l'absence de cytokines indicatrices d'une stimulation lymphocytaire.
